# EUROPEAN PATENT SPECIFICATION

(11) **EP 3 351 551 B1**
(45) Date of publication and mention of the grant of the patent: **07.12.2022**
(21) Application number: 16845696.0
(22) Date of filing: 13.09.2016
(51) Int. Cl.: C07H 19/10, C07H 1/00, C07F 9/24, C07H 1/02

(54) **PREPARATION METHOD OF NUCLEOSIDE PHOSPHORAMIDATE PRODRUGS AND INTERMEDIATES THEREOF**
VERFAHREN ZUR HERSTELLUNG VON NUKLEOSID-PHOSPHORAMIDAT-PRODRUGS UND ZWISCHENPRODUKTE DAVON
PROCÉDÉ DE PRÉPARATION DE PROMÉDICAMENTS DE PHOSPHORAMIDATE DE NUCLÉOSIDE ET PRODUITS INTERMÉDIAIRES CORRESPONDANTS

(30) Priority: 16.09.2015 CN 201510588122
(43) Date of publication of application: 25.07.2018
(73) Proprietor: BrightGene Bio-Medical Technology Co., Ltd., Suzhou Industrial Park Suzhou Jiangsu 215123 (CN)
(72) Inventor: YUAN, Jiandong, Suzhou Jiangsu 215123 (CN); HUANG, Yangqing, Suzhou Jiangsu 215123 (CN); MIAO, Linfeng, Suzhou Jiangsu 215123 (CN); GU, Jianing, Suzhou Jiangsu 215123 (CN); LIANG, Chaohua, Suzhou Jiangsu 215123 (CN); WANG, Zhengye, Suzhou Jiangsu 215123 (CN); SUN, Zhanli, Suzhou Jiangsu 215123 (CN)
(74) Representative: Grünecker Patent- und Rechtsanwälte PartG mbB
(86) International application number: PCT/CN2016/098846
(87) International publication number: WO 2017/045582

(56) References cited:
- EP-A1- 2 752 422
- WO-A1-2012/075140
- WO-A1-2013/187978
- WO-A1-2014/033617
- WO-A1-2014/058801
- WO-A1-2014/076490
- WO-A1-2015/198058
- WO-A1-2015/198059
- WO-A1-2016/055769
- WO-A2-2005/012327
- US-A1- 2013 288 997
- MAGDALENA SLUSARCZYK ET AL: "Application of ProTide Technology to Gemcitabine: A Successful Approach to Overcome the Key Cancer Resistance Mechanisms Leads to a New Agent (NUC-1031) in Clinical Development", JOURNAL OF MEDICINAL CHEMISTRY, vol. 57, no. 4, 14 February 2014 (2014-02-14), pages 1531-1542, XP055568914, US ISSN: 0022-2623, DOI: 10.1021/jm401853a
- BRUCE S. ROSS ET AL: "Synthesis of Diastereomerically Pure Nucleotide Phosphoramidates", THE JOURNAL OF ORGANIC CHEMISTRY, vol. 76, no. 20, 21 October 2011 (2011-10-21), pages 8311-8319, XP055137289, ISSN: 0022-3263, DOI: 10.1021/jo201492m
- UGO PRADERE ET AL: "Synthesis of Nucleoside Phosphate and Phosphonate Prodrugs", CHEMICAL REVIEWS, vol. 114, no. 18, 24 September 2014 (2014-09-24), pages 9154-9218, XP055203528, ISSN: 0009-2665, DOI: 10.1021/cr5002035

## Description

### TECHNICAL FIELD

The present disclosure relates to the field of medical technology, and particularly to a method of preparing nucleoside phosphoramidate prodrugs and the intermediates thereof.

### BACKGROUND

NUC-1031 is a prodrug of gemcitabine developed by NuCana BioMed Ltd., and is currently in Phase II clinical trials for the treatment of cancers such as advanced solid tumors, pancreatic cancer and breast cancer. The CAS of NUC-1031 is 840506-29-8, and NUC-1031 has the structure as shown in the following formula 1, the following formulae Rₚ-1 and Sₚ-1 are the enantiomers at phosphorus atom P of NUC-1031, respectively:

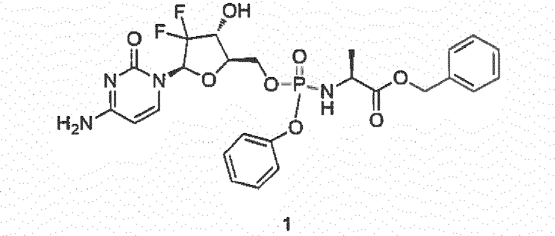

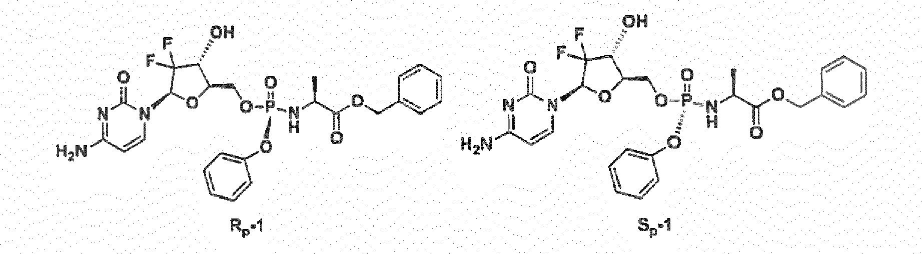

On page 70 in the description of WO2005012327 are disclosed the specific structure of NUC-1031 and the preparation method thereof, the method comprising, specifically, in the presence of NMI, reacting gemcitabine with benzyl-(benzoyl-L-alanine)-chlorophosphate at a molar ratio of 1:3 for 2 hours, with THF/pyridine as the solvent, to give a crude product, which is then subjected to separation and purification on a silica gel column, and eluted with dichloromethane/methanol (95:5) to give a foamy solid NUC-1031 with a yield of only 16%.

WO2014076490A1 discloses the following method of preparing NUC-1031,

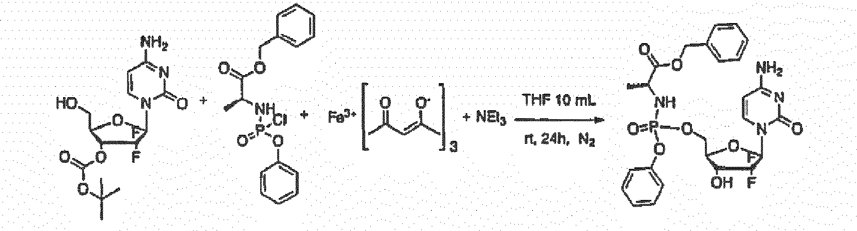

3'-Boc-protected gemcitabine (100 mg) is reacted with 2 mol equivalents of phenyl(benzyl-L-alanine)chlorophosphate (150 mg), with 0.5 mol equivalents of tris(acetylacetone)Fe(III) (56 mg) as the catalyst, 1.5 mol equivalents of DIPEA (55 µL) as the base, and 10 ml of THF as the solvent; the reaction is conducted at room temperature under the protection of nitrogen gas for 24 hours with a yield of 45%, wherein the ratio of the isomers, i.e., Rₚ:Sₚ is 3:1.

A literature entitled "Application of ProTide Technology to Gemcitabine: A Successful Approach to Overcome the Key Cancer Resistance Mechanisms Leads to a New Agent (NUC-1031) in Clinical Development" (Journal of Medicinal Chemistry, Volume 57, Issue 4, Pages 1531-1542) reported a method for preparing NUC-1031 by deprotecting the hydroxyl group of the compound 5f represented by the following formula in the presence of TFA with dichloromethane as the solvent and finally by purification on a silica gel column, the yield is 70%, the contents of the isomers are 48% and 52%, respectively;

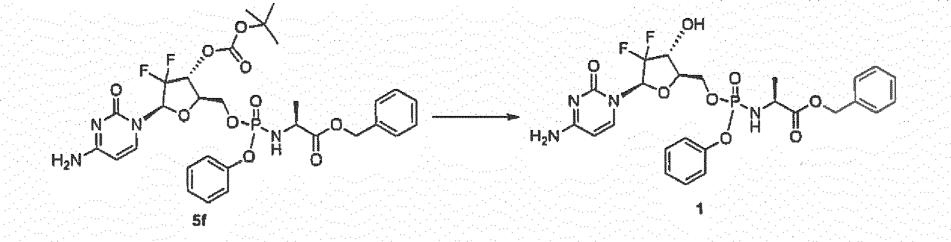

At present, the preparation method of the single isomer of NUC-1031 has not been reported in the prior art. Since the two enantiomers of the chiral P in the molecular structure of NUC-1031 are very similar in structure and polarity, it becomes very difficult to isolate a high-purity single isomer from the racemic mixture of NUC-1031, and in particular, it is even more difficult to balance purity and yield simultaneously in the purification process. The inventors have attempted to separate the isomers of NUC-1031 by various conventional methods in the art such as crystallization, silica gel column chromatography, reversed phase C18 silica gel-bonded preparative chromatography, normal phase preparative chromatography with spherical silica based packing, and resolution by chiral column, but all of them cannot isolate a single isomer with a purity of not less than 90%.

It is known in the art that usually when a racemic compound is used as a drug, the relationship between the configuration of the enantiomers and the pharmacological effect can be divided into the following scenarios: (I) the pharmacological effect of the drug is completely or mainly generated by one of the enantiomers, for example, (S)-naproxen has an analgesic effect 35 times stronger than that of the T isomer; (2) the two isomers have completely opposite pharmacological effects, for example, the dextrorotatory isomer of Picenadol is an opiate receptor agonist, while the levorotatory isomer thereof is an opiate receptor antagonist; (3) an isomer arises serious adverse reactions, for example, the emetic reaction of the anthelmintic tetramisole is caused by the dextrorotatory isomer thereof; (4) one pharmacological effect has high stereoselectivity, while the other pharmacological effects have low or no stereoselectivity, for example, addiction to the antitussive methorphan is mainly caused by the levorotatory isomer thereof, but in terms of the antitussive effect, the dextrorotatory isomer and the levorotatory isomer have the same intensity; and (5) the two enantiomers have different pharmacological effects, but the combination use is beneficial, for example, the dextrorotatory isomer of the antihypertensive nebivolol is a β-receptor blocker, while the levorotatory isomer thereof can reduce peripheral vascular resistance and has protective effect for heart.

Isolating a single isomer (Rₚ-1) or (Sₚ-1) from NUC-1031, studying its biological activities respectively, and studying the pharmacological effects, toxic side effects or adverse reactions of NUC-1031 to develop similar drugs with better activity and less toxic side effects will be of great significance. Preparing high-purity single isomer (Rₚ-1) or (Sₚ-1) that can meet the needs of clinical research is still urgent problem that needs to be solved in the art at present.

### Summary

The subject matter of the application is as set out in the appended claims. Any reference hereinafter to: "the present disclosure" must be understood as being given for comparative purposes.

One aspect of the invention relates to a method of preparing a composition, comprising the compound Sₚ-1 in an amount of at least about 95% by weight 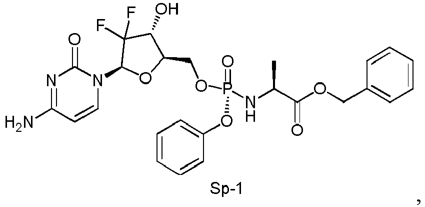 wherein the method comprises reacting a compound 61501c with a compound 61501b to produce a compound 61501a: 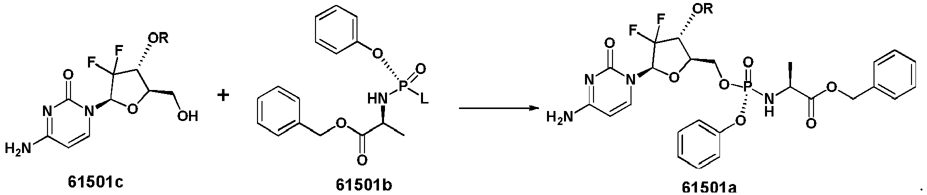
wherein R is H or a hydroxyl protecting group; L is halogen, aryloxide, benzenesulfonate group, camphorsulfonate group, or aryloxide substituted with at least one electron withdrawing group a leaving group;
optionally, when R is not H, the hydroxyl protecting group of the compound 61501a is deprotected to give the compound Sₚ-1.

Preferred embodiments are set forth in the subclaims.

Preferably, said electron withdrawing group is nitro or halogen. More preferably, L is nitrophenoxide, p-chlorophenoxide, o-chlorophenoxide, 2,4-dinitrophenoxide or pentafluorophenoxide.

In the above method, preferably, the hydroxyl protecting group R is alkylsilyl, alkyl or substituted alkyl, acyl or substituted acyl, alkoxycarbonyl or substituted alkoxycarbonyl. Further, said hydroxyl protecting group R is tetrahydropyranyl, benzyl, p-methylbenzyl, acetyl, propionyl, butyryl, benzoyl, *tert-*butyloxy carbonyl (Boc), benzyloxycarbonyl (Cbz), 9-fluorenylmethoxycarbonyl (Fmoc), *tert*-butyldimethylsilyl, trimethylsilyl or dimethylphenylsilyl.

In the above method, preferably, R is H or tert-butyloxy carbonyl (Boc), and L is pentafluorophenoxide.

Wherein when R is H, the method of preparing the compound Sₚ-1 comprises: 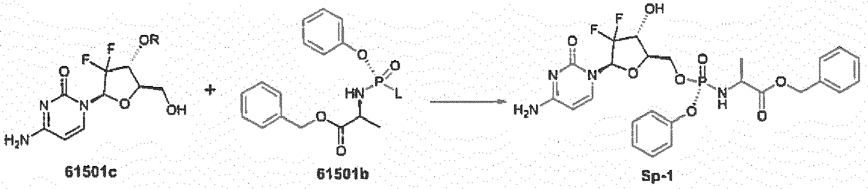
(I) reacting the compound 61501c (R=H) with the compound 61501b to give the compound Sₚ-1, wherein L is aryloxide, benzenesulfonate group, camphorsulfonate group, or aryloxide substituted with at least one electron withdrawing group.

Optionally, the method further comprises subjecting the compound Sₚ-1 to chromatographic analysis, extraction, or crystallization to give the purified Sₚ-1.

Wherein when R is a hydroxyl protecting group, the method of preparing the compound Sₚ-1 comprises: 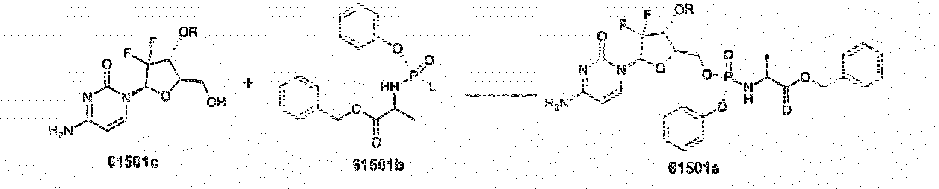
(1) reacting the compound 61501c (R is a hydroxyl protecting group) with the compound 61501b to give the compound 61501a; and
(2) deprotecting the hydroxyl protecting group of the compound 61501a to give the compound Sₚ-1.

Optionally, the method further comprises subjecting the compound Sₚ-1 to chromatographic analysis, extraction, or crystallization to give the purified Sₚ-1.

Wherein L is aryloxide, benzenesulfonate group, camphorsulfonate group, or aryloxide substituted with at least one electron withdrawing group.

Wherein said "hydroxyl protecting group" refers to a group that can combine with a hydroxyl group to form ester, silicyl oxide, or alkyl ether, including alkylsilyl , alkyl or substituted alkyl, and acyl or substituted acyl. For example, hydroxyl protecting groups that can combine with a hydroxyl group to form esters include but are not limited to C₁₋₆ alkylcarbonyl such as formyl, acetyl, chloroacetyl, butyryl, propionyl and the like; and substituted carbonyl such as tert-butyloxy carbonyl (Boc), benzyloxycarbonyl (Cbz), 9-fluorenylmethoxycarbonyl (Fmoc) and the like. Said hydroxyl protecting groups that form silicyl oxide include but are not limited to trifluoromethylsilyl, trimethylsilyl, dimethylphenylsilyl, *tert*-butyl dimethylsilyl and the like. Said hydroxyl protecting groups that can form alkyl ethers with a hydroxyl group include but are not limited to C₁₋₆ alkyl such as methyl, ethyl, propyl, butyl, tert-butyl and the like, and substituted C₁₋₆ alkyl such as benzyl, p-methylbenzyl, triphenylmethyl, and tetrahydropyranyl.

In the above method, the protecting group R of the compound 61501a is deprotected according to a conventional method for deprotecting a hydroxyl protecting group in this field to give the compound Sₚ-1. For example, when R is benzyl or substituted benzyl, Pb-C/H₂ catalytic hydrogenation can be selected to deprotect the protecting group. The reaction solvent is preferably tetrahydrofuran (THF), methanol, toluene, hexane or the like. When R is alkylsilyl (e.g., trimethylsilyl), it is preferable to deprotect the alkylsilyl protecting group in an organic solvent (e.g., HCl-MeOH, HCl-dioxane system, or AcOH-THF system) under acidic conditions, or to deprotect the alkylsilyl protecting group by using tetraalkylammonium fluoride. When R is alkylacyl (e.g., acetyl, propionyl, butyryl, etc.), deprotection via hydrolysis under acidic or alkaline conditions can be selected, for example, the corresponding alkylacyl can be deprotected by stirring at room temperature using methanol as a solvent under the conditions of sodium methoxide.

In the above method, it is further preferred that the hydroxyl protecting group R is tert-butyloxy carbonyl (Boc). It is preferred that the compound 61501a, under the atmosphere of nitrogen gas, is subjected to catalysis with an acid (e.g., trifluoroacetic acid, hydrogen chloride/ethyl acetate) in a suitable solvent (e.g., dichloromethane, ethyl acetate) and at a suitable temperature (e.g., -10°C to 5°C, preferably 0°C) to deprotect the hydroxyl protecting group. After the completion of the reaction, an aqueous solution of a weak alkali (e.g., a saturated solution of sodium bicarbonate) and an organic solvent (e.g., ethyl acetate) are added, and then, the organic phase is separated, concentrated and dried to give the crude compound Sₚ-1. It is further preferred to purify the above crude product, for example, by using silica gel column chromatography (with dichloromethane-methanol as the eluting reagent) so as to prepare the high-purity compound Sₚ-1.

In the above method, the compound 61501b is as follows, 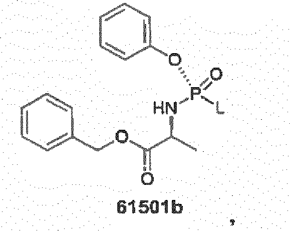 wherein L is aryloxide, benzenesulfonate group, camphorsulfonate group, or aryloxide substituted with at least one electron withdrawing group. Preferably, said electron withdrawing group is nitro or halogen.

Further more preferably, L is nitrophenoxide, p-chlorophenoxide, o-chlorophenoxide, 2,4-dinitrophenoxide or pentafluorophenoxide.

In the above method of preparing the compound Sₚ-1, more preferably, R is H or tert-butyloxy carbonyl (Boc), and L is pentafluorophenoxide.

On the other hand, the present disclosure also provides a composition obtained by any one of the foregoing methods, said composition comprises the compound Sₚ-1 in an amount of at least about 95% by weight of said composition.

Further, the present disclosure also provides a composition obtained by any one of the foregoing methods, said composition comprises the compound Sₚ-1 in an amount of at least about 99% by weight of said composition.

In the present disclosure, the compound 61501c (when R is a hydroxyl protecting group) is commercially available or can be prepared according to the method of a conventional hydroxyl protecting reaction, for example, the compound 61501c (when R is a hydroxyl protecting group) can be obtained from a compound 61501d:

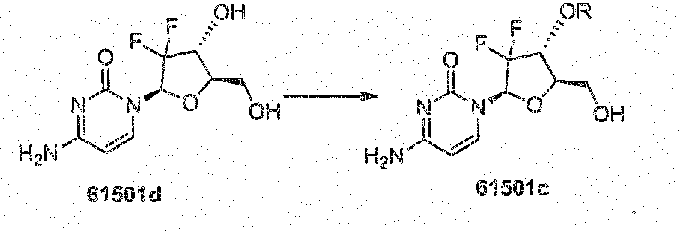

Reaction condition that is conventional in this field is selected to conduct the reaction according to different hydroxyl protecting groups R. For example, when R is a silicyl oxide-based protecting group (trimethylsilyl, *tert*-butyldimethylchlorosilyl, *tert*-hexyldimethylchlorosilyl, etc.), it is usually required to react in the presence of alkaline at room temperature. When R is substituted alkyl such as benzyl or p-methylbenzyl, it is usually required to react by reflux in the presence of alkaline using acetonitrile or acetone as the solvent. When R is acyl or substituted acyl (e.g., tert-butyloxy carbonyl, chloroacetyl, acetyl, etc.), it is usually required to react in the presence of alkaline at room temperature.

As an example, when R is *tert*-butyloxy carbonyl, it is preferred that the compound 61501d is reacted with di-tert-butyl dicarbonate in a suitable solvent (e.g., tetrahydrofuran, dichloromethane, isopropanol or a mixed solution of one of them with water) under alkaline (e.g., sodium carbonate, sodium bicarbonate, etc.) conditions.

On the other hand, the present disclosure provides a method of preparing the compounds 61501b and 61501e, which comprises the following steps: 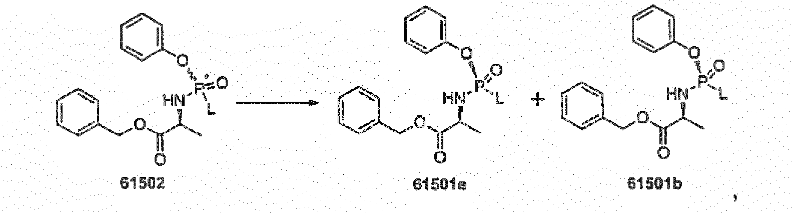
(1) reacting a compound (PhO)P(O)(L')₂ and alanine benzyl ester in the presence of a first alkali to obtain (L')P(O)(PhO)(Ala-CH₂Ph);
(2) reacting (L')P(O)(PhO)(Ala-CH₂Ph) with a phenol in the presence of a second alkali to obtain a mixture comprising the compounds 61501b and 61501e; and
(3) subjecting the mixture comprising the compound 61501b and the compound 61501e in step (2) to extraction, chromatographic separation or crystallization to obtain the compound 61501b or the compound 61501e;
wherein L is a leaving group, said leaving group includes aryloxide, benzenesulfonate group, camphorsulfonate group, or aryloxide substituted with at least one electron withdrawing group. L' is a leaving group independent of L.

Alanine benzyl ester exists in its hydrochloride form; said first alkali and the second alkali are independently selected from organic alkalis or inorganic alkalis. Said organic alkalis include but are not limited to triethylamine, DIPEA, NMM, pyridine and piperidine; said inorganic alkalis include but are not limited to sodium carbonate, sodium bicarbonate, potassium carbonate, potassium bicarbonate, and the like.

Said leaving group has the same meaning as known to those skilled in the art (Advanced Organic Chemistry: reactions, mechanisms and structure (4th ed., edited by Jerry March, John Wiley and Sons, 1992, pp. 351-357), and it indicates a group which is a part of a substrate molecule and connects thereto. In reactions where the substrate molecule undergoes a substitution reaction (e.g., a nucleophile), the leaving group is subsequently substituted. Examples of leaving groups include but are not limited to halogen (F, Cl, Br, or I), preferably Cl, Br or I; tosylate group, mesylate group, triflate group, acetate group, camphorsulfonate group, aryloxide and aryloxide substituted with at least one electron withdrawing group (e.g., p-nitrophenoxide, 2-chlorophenoxide, 4-chlorophenoxide, 2,4-dinitrophenoxide, pentafluorophenoxide, etc.) and the like.

As an example, when L is pentafluoroaryloxide, a compound 61501h is reacted with a compound 61501g in the presence of alkaline and then reacted with a compound 61501f in the presence of alkaline to give a mixture of the compounds 61501e and 61501b:

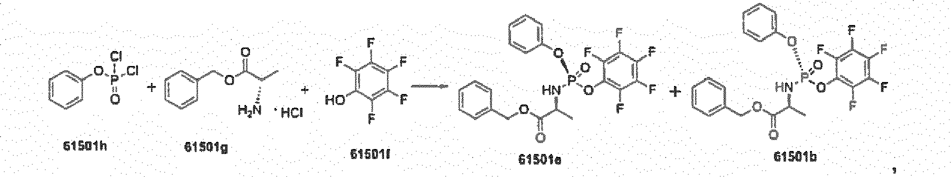

In the above step, the reaction is preferably carried out under the protection of N₂, the compound 61501h is added to a suitable solvent (e.g., dichloromethane, isopropanol, dimethylformamide (DMF), dioxane, etc.), and then the compound 61501g and a suitable base (e.g., triethylamine, N,N-diisopropylethylamine (DIPEA), N-methylmorpholine (NMM), pyridine and piperidine) are added at a suitable temperature (preferably -80°C); after the dropwise addition is complete, it is preferred that the reaction is carried out at room temperature overnight and then, to the reaction solution, the compound 61501f and a suitable base (such as triethylamine, DIPEA, NMM, pyridine and piperidine) are added; after the reaction is complete, the solvent is removed by distillation, ethyl acetate and water are added for extraction, and a mixture of the target compounds 61501e and 61501b is obtained by separation.

Further, in the above method, the mixture comprising the compound 61501b and the compound 61501e is dissolved or suspended into a solvent in step (3). Then, an anti-solvent is added to crystalize and obtain the compound 61501b.

In a preferred embodiment of the present disclosure, the compound 61501b is isolated by crystallization, which comprises a first step of dissolving the compound 61502 in an organic solvent and stirring for dissolution; and a second step of adding an anti-solvent dropwise to the above solution system to crystallize and obtain the compound 61501b.

Wherein said organic solvent comprises at least one of the following: C₁₋₈ alcohols, C₂₋₈ ethers, C₃₋₇ ketones, C₃₋₇ esters, C₁₋₂ chlorocarbons, and C₂₋₇ nitriles. Further preferably, said organic solvent is selected from ethyl acetate, tert-butyl methyl ether, isopropanol or tetrahydrofuran.

Wherein said anti-solvent comprises at least one of the following: C₅₋₁₂ saturated hydrocarbons, C₆₋₁₂ aromatic hydrocarbons, and petroleum ether. More preferably, said anti-solvent is selected from petroleum ether or hexane.

Further, in the above method, the volume ratio of the organic solvent to the anti-solvent is 1:2 to 10 (v/v), preferably 1:4 to 6 (v/v). The amount ratio of the compound 61502 to that of the organic solvent is 1:1 to 10 (w/v), preferably 1:1.25 to 2.5 (w/v).

In another preferred embodiment, said organic solvent is selected from ethyl acetate, and said anti-solvent is selected from petroleum ether.

More preferably, in the above method, said crystallization method comprises a first step of dissolving the compound 61502 in ethyl acetate and stirring for dissolution at room temperature; a second step of adding petroleum ether dropwise to the above solution system, crystallizing, filtering, thereby giving the compound 61501b. Preferably, the amount ratio of the compound 61502 to that of ethyl acetate is 1:1.25 to 2.5 (w/v), and the amount ratio of ethyl acetate to that of petroleum ether is 1:4 to 6 (v/v).

In another preferred embodiment, the temperature of stirring for dissolution and crystallizing is 10°C to 50°C, more preferably, the temperature is 25°C to 30°C.

Further, in the above method, the mixture comprising the compound 61501b and the compound 61501e is subjected to separation through a preparative chromatography column to obtain the compound 61501e in step (3).

In another preferred embodiment of the present disclosure, the method of preparing the compound 61501e comprises subjecting the compound 61502 to preparative liquid chromatography to separate and obtain the compound 61501e.

Preferably, the mobile phase in said preparative liquid chromatography is methanol-glacial acetic acid/water.

Further, it is preferred that said preparative liquid chromatography uses a column with a C18 filler of 10µm as the stationary phase to prepare the compound 61501e; more preferably, after the compound 61502 is dissolved in methanol and pass through a C18 (10µm) reversed phase column, the compound 61501e is obtained by gradient elution using methanol-5‰ acetic acid/water (the proportion of methanol is 70% to 85%) as the mobile phase.

In a more preferred embodiment, chromatographic conditions of the preparative liquid chromatography are as shown in the following Table I.

**Table I**

| Preparative column: 300DAC | | | 300×250mm | | filler: C18, 10µm | | |
|---|---|---|---|---|---|---|---|
| Mobile phase A: methanol | | | Mobile phase B: 5‰ acetic acid/water | | | | |
| Gradient conditions | Time (min) | Concentration of mobile phase A | | Concentration of mobile phase B | | Detection wavelength λ (nm) | Flow rate V (L/min) |
| | 0 | 70 | | 30 | | 260 | 1.8 |
| | 30 | 70 | | 30 | | 260 | 1.8 |
| | 100 | 85 | | 15 | | 260 | 1.8 |

Further, the present disclosure provides the following compounds:

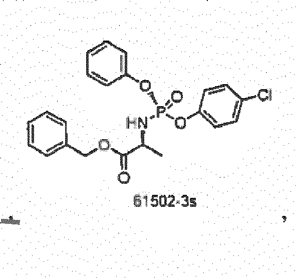

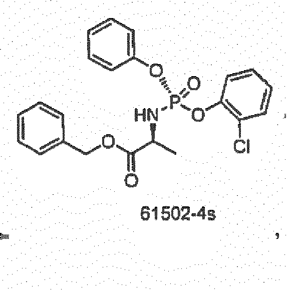

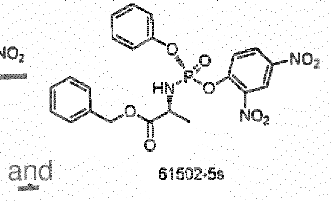

The following definitions and terms are used herein and these terms are well known to those skilled in the art. Unless otherwise specified, these definitions are used throughout the description and claims. Chemical names, common names, and chemical structures are used interchangeably to describe the same structure. These definitions apply regardless of whether a certain term is used alone or in combination with other terms. Therefore, the definition of "alkyl" not only applies to "alkyl" but also applies to the alkyl moiety of "hydroxyalkyl", "haloalkyl", "alkoxy" and the like. Unless otherwise well-known, specified or proven to be the contrary, the connection point of a multiple-term substituent (a single substituent determined by combining two or more terms) with the subject structure is the last-mentioned term of the multiple-term substituent. For example, a cycloalkylalkyl substituent is attached to the target structure (e.g., structure-alkyl-cycloalkyl) via an "alkyl" moiety.

The term "alkyl" refers to a linear or branched saturated monovalent hydrocarbon residue containing 1 to 30 carbon atoms. The term "C₁₋₆ alkyl" refers to an alkyl group containing 1 to 6 carbon atoms. Examples of an alkyl group include but are not limited to lower alkyl including methyl, ethyl, propyl, isopropyl, n-butyl, isobutyl, tertiary butyl or pentyl, isopentyl, neopentyl, hexyl, heptyl, and octyl. The term "substituted alkyl" refers to an alkyl which may be substituted with one or more substituents independently selected from halogen, aryl, cycloalkyl, cyano, hydroxy, alkoxy, carboxyl and -C(O)O-alkyl.

The term "alkoxyl" refers to an alkyl-O-group in which the alkyl is as described above. A preferred alkoxyl contains 1 to 12 carbon atoms, preferably 1 to 6 carbon atoms. Suitable alkoxyls include but are not limited to methoxyl, ethoxyl and isopropoxyl. The alkyl in an alkoxyl is connected to an adjacent moiety via the ether oxygen.

The terms "halogen" and "halide" refer to chlorine, bromine, iodine or fluorine atom groups. Chlorides, bromides, iodides and fluorides are the preferred halides.

The term "aryl" refers to a substituted or unsubstituted aromatic, monocyclic or bicyclic chemically feasible carbocyclic ring system having 1 to 2 aromatic rings. The aryl moiety usually has 6 to 14 carbon atoms. Representative examples include but are not limited to phenyl, tolyl, xylyl, naphthyl and the like. If desired, the carbocyclic moiety may be substituted with I to 5 substituents, preferably 1 to 3 substituents, said substituents include halo, alkyl, phenyl, hydroxy, alkoxy, amino and the like.

The term "acyl" refers to a substituent containing a carbonyl moiety and a non-carbonyl moiety. The carbonyl moiety contains a double bond between the carbonyl carbon and the heteroatom, wherein the heteroatom is selected from O, N and S. When the heteroatom is N, N is substituted with a lower alkyl. The non-carbonyl moiety is selected from: linear, branched and cyclic alkyl including but not limited to linear, branched or cyclic C₁₋₂₀ alkyl, C₁₋₁₀ alkyl or lower alkyl; alkoxyalkyl including methoxymethyl; aralkyls including benzyl; aryloxidealkyls, such as phenoxymethyl; or aryl including phenyl optionally substituted with the following: halogen (F, Cl, Br, I), hydroxy, C₁₋₄ alkyl or C₁₋₄ alkoxyl, sulfonate group such as alkyl- or aralkyl-sulfonyl including methanesulfonyl, mono-, di- or triphosphate group, trityl or monomethoxytrityl, substituted benzyl, trialkylsilyl (e.g., tert-butyldimethylsilyl) or diphenylmethylsilyl. When at least one aryl exists in the non-carbonyl moiety, the aryl preferably includes phenyl. The term "substituted acyl" refers to an acyl substituted with one or more substituents independently selected from halogen, aryl, cycloalkyl, cyano, hydroxy, alkoxy, carboxyl and -C(O)O-alkyl.

The term "electron withdrawing group" has its usual meaning here. Examples of an electron withdrawing group include but are not limited to halogen, -NO₂, -C(O)-(lower alkyl), -C(O)-(aryl), -C(O)O-(lower alkyl), -C(O)O-(aryl) and the like.

The term "P^{∗}" means that the phosphorus atom is chiral, and it has a corresponding Cahn-Ingold-Prelog designation of "R" or "S" which have their commonly accepted meanings.

On the other hand, the present specification also discloses the use of said compounds Rₚ-1 and Sₚ-1 in preparing a drug for the treatment of cancer; further, said cancer includes pancreatic cancer, advanced solid tumors, ovarian tumor, and breast cancer.

The present specification also discloses a pharmaceutical composition comprising the compound (Rₚ-1) or the compound (Sₚ-1) as a pharmaceutically active ingredient, said pharmaceutical composition also comprises at least one pharmaceutically acceptable excipient.

Said pharmaceutical composition can be administered orally or parenterally, including intravenously, subcutaneously, intraperitoneally, intramuscularly, by inhalation, rectally, and topically (e.g., buccally or sublingually).

Among them, pharmaceutical compositions for oral administration include tablets, capsules, granules or suspensions; the tablets for oral administration comprise the composition provided by the present disclosure as an active ingredient, and may further comprise one or more pharmaceutically acceptable excipients, such as diluent, disintegrant, binder, lubricant, sweetener, flavoring agent, pigment and preservative. When corn starch and alginic acid are used as disintegrants, suitable inert diluents include sodium carbonate, calcium carbonate, sodium phosphate, calcium phosphate and lactose. The binders include starch and gelatin, and optionally, the lubricant is magnesium stearate, stearic acid or talc. Optionally, said tablets may also be coated with glycerol monostearate or glycerol distearate to delay the absorption in stomach.

The capsules for oral administration include hard capsules and soft capsules, wherein the hard capsules comprise the pharmaceutical composition provided by the present disclosure as the effective active ingredient and a solid diluent; and the soft capsules comprise the pharmaceutical composition provided by the present disclosure as the effective active ingredient, and water or oil (e.g., peanut oil, liquid paraffin or olive oil).

The dosage form for rectal administration is suppository, wherein the base of the suppository may be cocoa butter or salicylate.

The forms of the formulations for vaginal administration include pessaries, tampons, creams, gels, pastes, foams, or sprays, wherein said formulations comprise a pharmaceutical composition containing the active ingredient and a conventional carrier known in the art.

When used in the dosage forms for intravenous injection, intraperitoneal administration, subcutaneous administration and intramuscular administration, the pharmaceutical compositions provided by the present disclosure are usually sterile solutions or sterile suspensions, and have suitable pH values and osmotic pressures. Such formulations can be prepared and obtained according to the conventional methods generally known in the art.

The administration dosage of said pharmaceutical composition is 0.1 to 300 mg/kg body weight/day; preferably 0.5 mg/kg body weight/day; and a further preferred suitable administration dosage is 1 to 50 mg/kg body weight/day, and more preferably I to 50 mg/kg body weight/day. Preferably, the composition is administered twice, three times, four times, or five times a day at intervals. The composition preferably comprises 10 to 1500 mg of the active ingredient, more preferably 20 to 1000 mg of the active ingredient, and most preferably 50 to 700 mg of the active ingredient as one dosage unit.

In the composition provided by the present disclosure, the diastereoisomeric Sₚ-1 is highly enriched, which provides starting material for further conducting clinical research on the pharmacological activity of NUC-1031 and developing new anticancer drugs with good activity and low side effects in the future. Besides, the inventors inventively carry out the separation of the single isomers right after the completion of the first-step reaction, which significantly saves the starting material as compared with the prior art that isolates the single isomers from racemic mixtures and/or racemates. Meanwhile, the separation of the isomers in the first-step reaction adopts the preparative liquid chromatography or crystallization method, the operation of which is convenient, and said method greatly saves costs as compared with the prior art that uses chiral chromatographic column for separation. In addition, the preparation method provided by the present disclosure has the advantages of simple and convenient operation of the entire route, low requirements for the equipment, high purity of the resultant product, and suitability for industrialized production.

### BRIEF DESCRIPTION OF THE DRAWINGS

Fig. 1 shows an HPLC analysis chromatogram of the compound 61501b prepared according to the method of Example 3, wherein the HPLC purity of the compound 61501b is 100%, and its peak data is shown as below:

| Peak # | Name | Retention time | Area | Peak height | Area% |
|---|---|---|---|---|---|
| I | 61501b | 26.628 | 566450 | 82468 | 100 |
| Total | | | 566450 | 82468 | 100 |

Fig. 2 is a ³¹P-NMR spectrum of the compound Rₚ-1 prepared according to the method of Example 10, in which one peak is at δ_{P} 3.81 (not according to the invention).
Fig. 3 shows an HPLC analysis chromatogram of the compound Sₚ-1 prepared according to the method of Example 20, wherein the HPLC purity of the compound Sₚ-1 is 100%, and its peak data is shown as below:

| Peak # | Retention time | Area | Peak height | Area% |
|---|---|---|---|---|
| | 8.388 | 5096820 | 317635 | 100 |
| Total | | 5096820 | 317635 | 100 |

Fig. 4 is a ³¹P-NMR spectrum of the compound Sₚ-1 prepared according to the method of Example 20, in which one peak is at δ_{P} 3.64.
Fig. 5 is ¹H-NMR spectrum of the compound Sₚ-1 prepared according to the method of Example 20.
Fig. 6 shows a. comparison diagram of the simulated XRPD of the single crystal of the compound Sₚ-1 with the reflected XRPD of the single crystal.
Fig. 7 is a photomicrograph of a single crystal of the compound Sₚ-1.
Fig. 8 shows a unit cell dimensions of the single crystal of the compound Sₚ-1.
Fig. 9 shows a stereostructure diagram of the molecule of the compound Sₚ-1.
Fig. 10 shows a stereostructure diagram of the molecule of the compound Sₚ-1.

### DETAILED DESCRIPTION

The contents of the present disclosure is further explained and described below in conjunction with the Examples. It should be understood that these Examples are merely used to describe the present disclosure but are not intended to limit the scope of the present disclosure. As for the experimental methods whose specific conditions are not indicated in the following Examples, the conditions are usually in accordance with the conventional conditions or the conditions suggested by the manufacturers. Unless otherwise specified, all percentages, rates, ratios, or parts are in terms of weight.

The units of the percent weight in volume in the present disclosure are well known to those skilled in the art and the unit of the percent weight in volume refers to, for example, the weight of a solute in 100 milliliter of a solution. Unless otherwise defined, all the professional and scientific terms used herein have the same meanings as is familiar to one skilled in the art. In addition, any methods and materials similar or equivalent to the described contents can be used in the method of the present disclosure. The preferred embodiments and materials described herein are for illustrative purposes only.

The purities of the intermediate compounds 61501e and 61501b of the present disclosure are determined by an HPLC method, which is conducted with the following column and conditions: YMC hydrosphere 150 mm × 4.6 mm, 3 µm; 40% to 85% methanol and 2‰ phosphoric acid/water are used as the mobile phases; run time: 46 min; gradient elution and adjusting the proportion of the mobile phase if necessary, flow rate: 1.0(ml/min).

In the present disclosure, the purities of the compound Rₚ-1 and Sₚ-1 are determined by an HPLC method, which is conducted with the following column and conditions: octadecylsilane-bonded silica is used as a filler (YMC-hydrosphere C18 column, 150 mm × 4.6 mm, 3 µm); a 0.1% phosphoric acid-methanol (10 to 50 : 90 to 50) serves as the mobile phase; the flow rate is 1.0 (ml/min), and the detection wavelength is 272 nm.

### Example 1 Preparation of the compound 61502

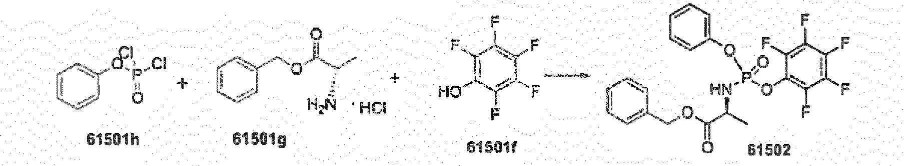

At -80°C, to a solution of 61501h (20g) in 60ml of dichloromethane, 20.6g of 61501g was added, and then 19.3g of triethylamine diluted in 20ml of dichloromethane was added. The mixture was stirred overnight at room temperature. To the mixture, 61501f was added, and then 19.3g of triethylamine diluted in 20ml of dichloromethane was added. The mixture was stirred at room temperature for 4h. The solvent was directly removed from the mixture, the residue was dissolved in 200ml of ethyl acetate and 400ml of water. After the ethyl acetate was separated, the aqueous phase was washed twice with ethyl acetate (2×100ml), each time with 100 ml of ethyl acetate. The ethyl acetate phases were combined, washed with saline, and dried over anhydrous sodium sulfate. Distilling off ethyl acetate resulted in the target compound 61502, which was used directly in the subsequent purification.

### Example 2 Preparation of the compound 61501e

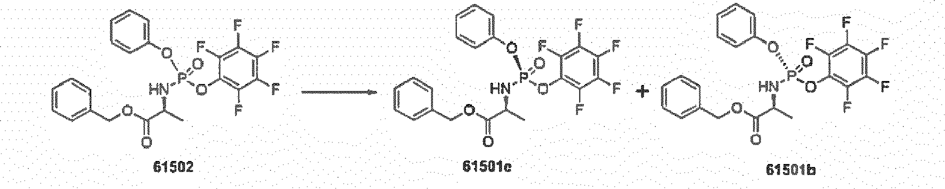

47g of the compound 61502 was dissolved with 470ml of methanol and purified and prepared through a preparative column with C18 filler (10µm) as the stationary phase, the preparation method was as the following Table I: methanol and 5‰ acetic acid/water served as the mobile phases respectively, the proportion of methanol increased from 70% to 85%, gradient elution.

**Table I: Preparation Method**

| Preparative column: 300DAC | | | 300×250mm | | | filler: C18, 10µm | | |
|---|---|---|---|---|---|---|---|---|
| Mobile phase A: methanol | | | | Mobile phase B: 5‰ acetic acid/water | | | | |
| Gradient conditions | Time (min) | Concentration of mobile phase A | | | Concentration of mobile phase B | | Detection wavelength λ(nm) | Flow rate V (L/min) |
| | 0 | 70 | | | 30 | | 260 | 1.8 |
| | 30 | 70 | | | 30 | | 260 | 1.8 |
| | 100 | 85 | | | 15 | | 260 | 1.8 |

| | | | | | | | | |
|---|---|---|---|---|---|---|---|---|
| Eluents were fractionally collected to give 12g of the compound 61501e with a yield of 28%; HPLC purity: 99.8%. | | | | | | | | |

### Example 3 Preparation of the compound 61501b

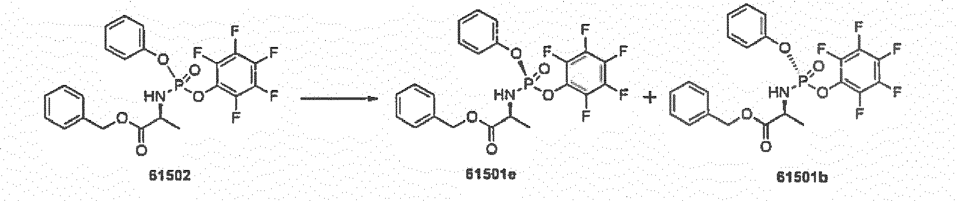

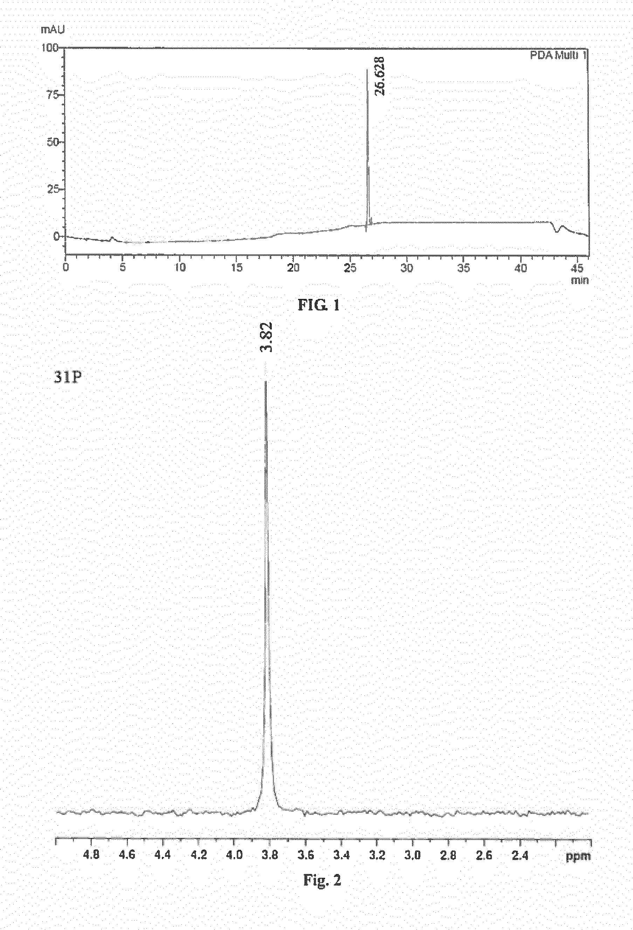

120g of the compound 61502 was dissolved with 240ml of ethyl acetate, and the mixture was stirred constantly, 720ml of petroleum ether was slowly added dropwise at room temperature, crystals precipitated and the filtrate was removed by filtration to obtain 48.8 g of the compound 61501b in total with a yield of 40.6%; HPLC purity: 100% (as shown in Fig. 1).

### Example 4 Preparation of the compound 61501c

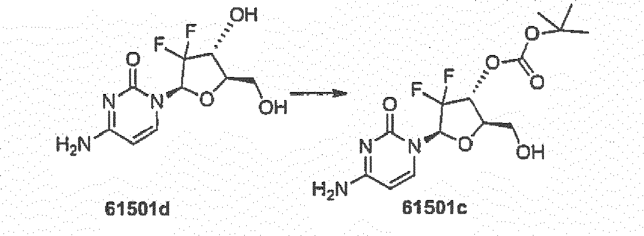

At room temperature, to a solution of the compound 61501d (20g) in a mixed solution of 200ml of tetrahydrofuran and 100ml of water, 35.4g of sodium carbonate was added, and then 17.5g of di-tert-butyl dicarbonate was added, and the mixture was stirred at room temperature until the reaction was complete. The mixture was extracted three times with ethyl acetate, each time with 200 ml of ethyl acetate. The ethyl acetate phases were combined, washed with saline and dried over anhydrous sodium sulfate. After the solvent was eliminated by distillation, 18g of the compound 61501c was obtained via silica gel column chromatography (mobile phase: methanol/dichloromethane (the proportion of methanol increased from 2.5% to 10%, gradient elution).

### Example 5 Preparation of a compound 61501c2

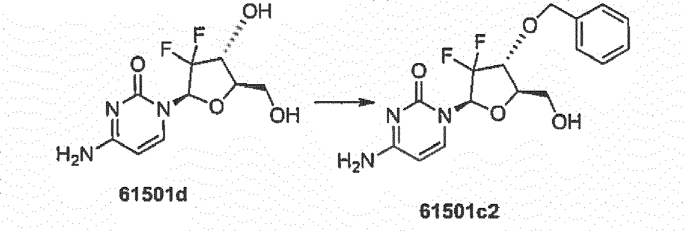

At room temperature, under the protection of nitrogen gas, 3g of potassium carbonate was added to acetonitrile in which 1g of 61501d and 1g of BnBr had been dissolved. The solution was warmed until reflux started, and the temperature was kept unchanged until the reaction was complete. The solvent was directly removed from the reaction solution, the residue was dissolved with 50ml of ethyl acetate, washed with saturated saline and dried over anhydrous sodium sulfate. After the solvent was removed, 900mg of the compound 61501c2 was obtained via silica gel column chromatography (mobile phase: methanol/dichloromethane (the proportion of methanol increased from 2.5% to 10%), gradient elution).

### Example 6 Preparation of a compound 61501c3

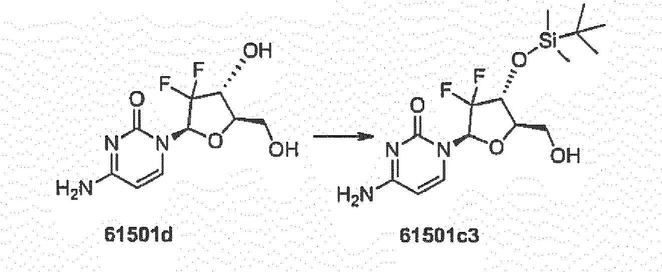

At room temperature, under the protection of nitrogen gas, 0.8g of tert-butyl dimethylchlorosilane was added to 10ml of pyridine in which 1g of 61501d had been dissolved, and the temperature was kept unchanged until the reaction was complete. The solvent was directly removed from the reaction solution, the residue was dissolved with 50ml of ethyl acetate, washed with saturated saline and dried over anhydrous sodium sulfate. After the solvent was removed, 500mg of the compound 61501c3 was obtained via silica gel column chromatography (mobile phase: methanol/dichloromethane (the proportion of methanol increased from 2.5% to 10%), gradient elution).

### Example 7 Preparation of a compound 61501c4

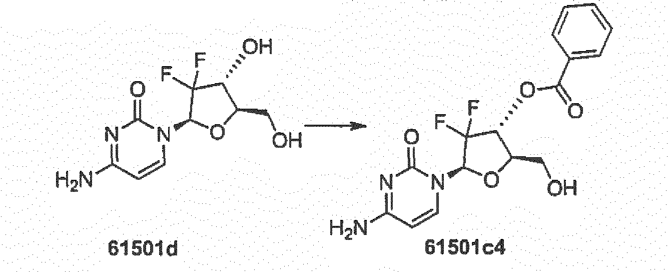

At room temperature, under the protection of nitrogen gas, 0.8g of BzCl was added to 10ml of pyridine in which 1g of 61501d had been dissolved, and the temperature was kept unchanged until the reaction was complete. The solvent was directly removed from the reaction solution, the residue was dissolved with 50ml of ethyl acetate, washed with saturated saline and dried over anhydrous sodium sulfate. After the solvent was removed, 600mg of the compound 61501c4 was obtained via silica gel column chromatography (mobile phase: methanol/dichloromethane (the proportion of methanol increased from 2.5% to 10%), gradient elution).

### Example 8 Preparation of the compound Rₚ-1 (Reference)

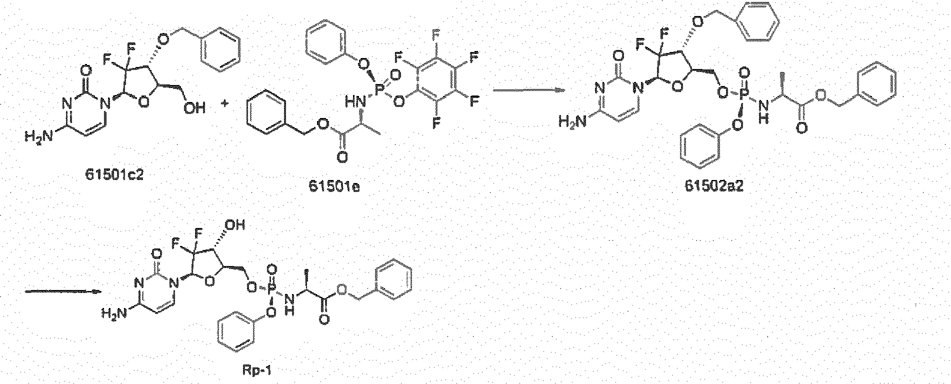

(1) Preparation of the compound 61502a2: To a solution of the compound 61501c2 (800mg) in 10ml of tetrahydrofuran was added a tert-butylmagnesium chloride solution (1.0mol/L, 6.5ml) at 0°C. After the mixture was stirred and reacted for 1 h, 2g of the compound 61501e was added, the mixture was stirred at room temperature until the reaction was complete. To the mixture, 50ml of water was added, and the aqueous phase was extracted three times with ethyl acetate, each time with 50ml of ethyl acetate. The ethyl acetate phases were combined, washed with saline, dried over anhydrous sodium sulfate, and the solvent was removed. 700mg of the compound 61502a2 was obtained by purification via silica gel column chromatography (mobile phase: methanol/dichloromethane (the proportion of methanol increased from 2.5% to 5%), gradient elution).
(2) Preparation of the compound Rₚ-1: 100mg of Pd/C was added to a solution of the compound 61502a2 (500mg) in 5ml of methanol at room temperature. After the reaction system was purged with hydrogen gas, the reaction proceeded at room temperature with the pressure maintained at 0.4bar until the reaction was complete. The reaction solution was directly filtered. After the solvent was removed from the filtrate, 240mg of the compound Rₚ-1 was obtained by purification via silica gel column chromatography (mobile phase: methanol/dichloromethane (the proportion of methanol increased from 2.5% to 10%), gradient elution), HPLC purity: 99.5%. ³¹P-NMR (202MHz, MeOD): δ_{P} 3.81. ¹H-NMR (500MHz, MeOD): δ_{H}: 7.56, 7.52 (2d,J=7.5Hz,1H,H-6), 7.38-7.33(m,7H,ArH), 7.26-7.19 (m,3H,ArH), 6.25 (apparent q, J=7.5Hz,1H,H-1'), 5.88, 5.84 (2×d,J=7.5Hz,1H,H-5), 5.18-5.12 (m,2H,OCH₃Ph), 4.49-4.42(m,1H,H-5'), 4.38-4.31 (m,1H,H-5'), 4.25-4.18 (m,1H,H-3'), 4.07-4.01 (m,2H,H-4',CHCH₃), 1.38 (apparent t,J=8.5Hz,3H,CHCH₃).

### Example 9 Preparation of the compound Rₚ-1 (Reference)

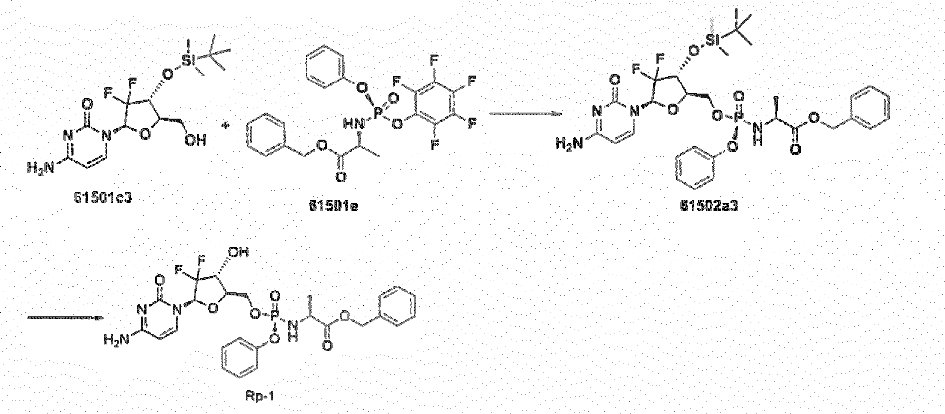

(1) Preparation of the compound 61502a3: To a solution of the compound 61501c3 (800mg) in 10ml of tetrahydrofuran, a tert-butylmagnesium chloride solution (1.0mol/L, 7ml) was added at 0°C. After the mixture was stirred and reacted for 1h, 2g of the compound 61501e was added and the mixture was stirred at room temperature until the reaction was complete. To the mixture, 50ml of water was added, and the aqueous phase was extracted three times with ethyl acetate, each time with 50ml of ethyl acetate. The ethyl acetate phases were combined, washed with saline, dried over anhydrous sodium sulfate, and the solvent was removed. 400mg of the compound 61502a3 was obtained by purification via silica gel column chromatography (mobile phase: methanol/dichloromethane (the proportion of methanol increased from 2.5% to 5%), gradient elution).
(2) Preparation of the compound Rₚ-1: At 0°C, to a solution of the compound 61502a3 (400mg) in 5ml of methanol, 1mol/L hydrochloric acid (1ml) was added, the reaction proceeded at 0°C until it was complete. The solvent was directly removed from the reaction solution, the residue was dissolved with 50ml of ethyl acetate, washed with saturated saline and dried over anhydrous sodium sulfate. After the solvent was removed, the compound Rₚ-1 (200mg) was obtained by purification via silica gel column chromatography (mobile phase: methanol/dichloromethane (the proportion of methanol increased from 2.5% to 10%), gradient elution), HPLC purity: 99.2%.

### Example 10 Preparation of the compound Rₚ-1 (Reference)

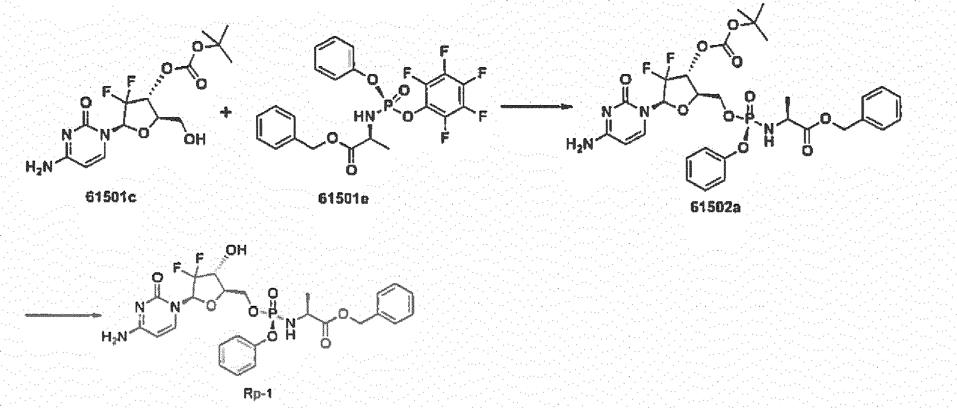

(1) Preparation of the compound 61502a: To a solution of the compound 61501c (2g) in 10ml of tetrahydrofuran was added a tert-butylmagnesium chloride solution (1.0mol/L, 16ml) at 0°C. After the mixture was stirred and reacted for 1h, 5g of the compound 61501e was added, the mixture was stirred at room temperature until the reaction was complete. To the mixture, 50ml of water was added, and the aqueous phase was extracted with ethyl acetate three times, each time with 100ml of ethyl acetate. The ethyl acetate phases were combined, washed with saline, dried over anhydrous sodium sulfate, and the solvent was removed. 2g of the compound 61502a was obtained by purification via silica gel column chromatography (eluting reagents: methanol/dichloromethane 2.5% to 5%) with a yield of 55%.
(2) Preparation of the compound Rₚ-1: 6ml of trifluoroacetic acid was added to a solution of the compound 61502a (2g) in 10ml of dichloromethane at 0°C, the mixture was stirred with the temperature kept unchanged until the reaction was complete. After the solvent was removed from the mixture, 50ml of sodium bicarbonate solution was added, then the resulted mixture was extracted three times with ethyl acetate, each time with 50ml of ethyl acetate. The ethyl acetate phases were combined, washed with saline and dried over anhydrous sodium sulfate. After the solvent was removed, 700mg of the compound Rₚ-1 was obtained via silica gel column chromatography (mobile phase: methanol/dichloromethane (the proportion of methanol increased from 2.5% to 5%), gradient elution), HPLC purity: 99.1%. ³¹P-NMR (202MHz,MeOD): δ_{P} 3.81 (as shown in Fig. 2). ¹H-NMR (500MHz,MeOD): δ_{H}: 7.56, 7.52 (2d,J=7.5Hz,1H,H-6), 7.38-7.33 (m,7H,ArH), 7.26-7.19 (m,3H,ArH), 6.25 (apparent q, J=7.5Hz, 1H,H-1'), 5.88, 5.84 (2×d,J=7.5Hz, 1H,H-5), 5.18-5.12 (m,2H,OCH₂Ph), 4.49-4.42 (m,1H,H-5'), 4.38-4.31 (m,1H,H-5'), 4.25-4.18 (m,1H,H-3'), 4.07-4.01 (m,2H,H-4',CHCH₃), 1.38 (apparent t,J=8.5Hz,3H,CHCH₃).

### Example 11 Preparation of the compound Rₚ-1 (Reference)

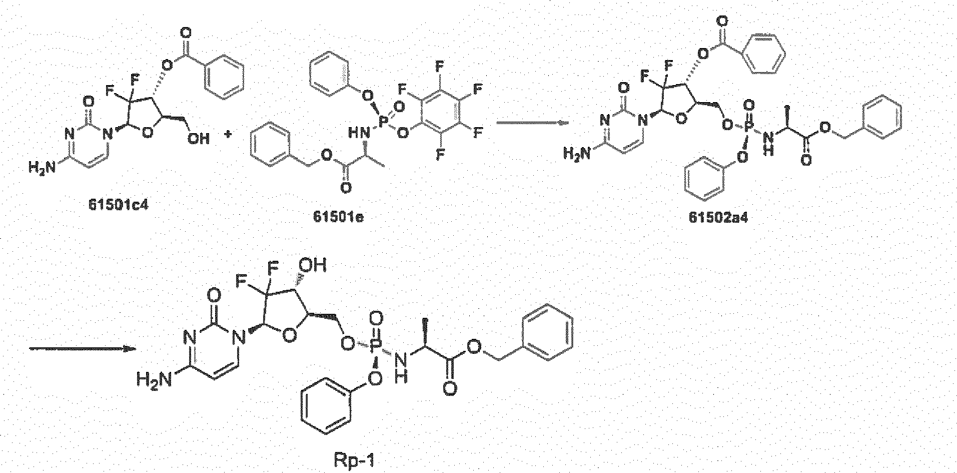

(1) Preparation of the compound 61502a4: To a solution of the compound 61501c4 (1g) in 10ml of tetrahydrofuran, a tert-butylmagnesium chloride solution (1.0mol/L, 8ml) was added at 0°C. After the mixture was stirred and reacted for 1h, 2.5g of the compound 61501e was added, and the mixture was stirred at room temperature until the reaction was complete. 50ml of water was added to the mixture, the aqueous phase was extracted with ethyl acetate three times, each time with 50ml of ethyl acetate. The ethyl acetate phases were combined, washed with saline, dried over anhydrous sodium sulfate, and the solvent was removed. 900mg of the compound 61502a4 was obtained by purification via silica gel column chromatography (mobile phase: methanol/dichloromethane (the proportion of methanol increased from 2.5% to 5%), gradient elution).
(2) Preparation of the compound Rp-1: To a solution of the compound 61502a4 (900mg) in 5ml of methanol, 1.5ml of 7mol/L methanolic ammonia solution was added at room temperature. The reaction proceeded at room temperature until it was complete. The solvent was directly removed from the reaction solution, the residue was dissolved with 50ml of ethyl acetate, washed with saturated saline, dried over anhydrous sodium sulfate, and then the solvent was removed. 500mg of the compound Rₚ-1 was obtained via silica gel column chromatography (mobile phase: methanol/dichloromethane (the proportion of methanol increased from 2.5% to 5%), gradient elution), HPLC purity: 99.5%.

### Example 12 Preparation of the compound Rₚ-1 (Reference)

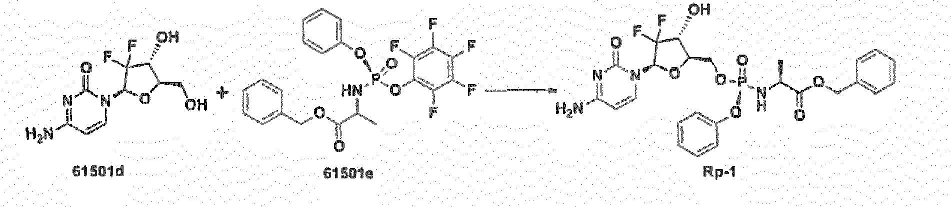

(1) Preparation of the compound Rₚ-1: To a solution of the compound 61501d (1g) in 10ml of tetrahydrofuran, a tert-butylmagnesium chloride solution (1.0mol/L, 8ml) was added at 0°C. After the mixture was stirred and reacted for 1h, 2.5g of the compound 61501e was added, and the mixture was stirred at room temperature until the reaction was complete. 50ml of water was added to the mixture, the aqueous phase was extracted three times with ethyl acetate, each time with 50ml of ethyl acetate. The ethyl acetate phases were combined, washed with saline, dried over anhydrous sodium sulfate, and the solvent was removed. 421mg of the compound Rₚ-1 was obtained by purification via silica gel column chromatography (mobile phase: methanol/dichloromethane (the proportion of methanol increased from 2.5% to 5%), gradient elution), HPLC purity: 99.5%.

### Example 13 Preparation of the compound 61502-1r and the compound 61502-1s

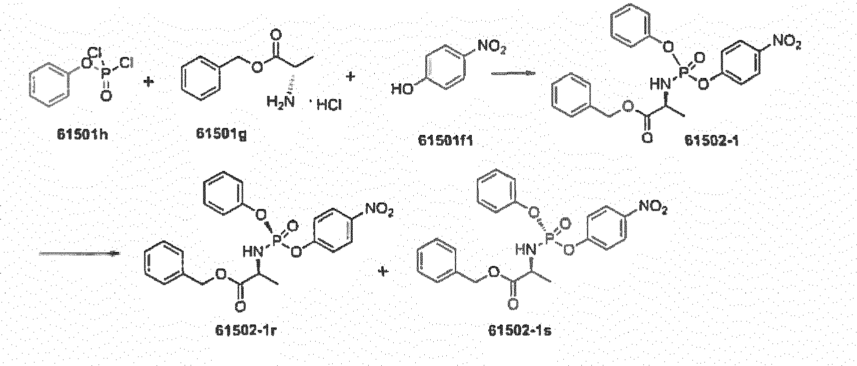

To a solution of 61501h (2g) in 10ml of dichloromethane, 2.1g of 61501g was added at -80°C, and then 2g of triethylamine diluted with 5ml of dichloromethane was added. The mixture was stirred overnight at room temperature. To the mixture was added 61501f1, and then 2g of triethylamine (diluted with 5ml of dichloromethane) was added. The mixture was stirred at room temperature for 4 h. The solvent was directly removed from the mixture, and the residue was dissolved in 50ml of ethyl acetate and 100ml of water. After the ethyl acetate was separated, the aqueous phase was washed twice with ethyl acetate, each time with 30ml of ethyl acetate. The ethyl acetate phases were combined, washed with saline, and dried over anhydrous sodium sulfate. Distilling off ethyl acetate resulted in the target compound 61502-1, which was used directly in the subsequent purification.

8g of the compound 61502-1 was dissolved with 50ml of methanol, and prepared and purified through a preparative column with C18 filler (10µm) as the stationary phase. The preparation method was as the following Table 1: methanol and 5‰ acetic acid/water served as the mobile phases respectively, the proportion of methanol increased from 70% to 80%, gradient elution.

**Table 1: Preparation Method**

| Preparative column: 100DAC | | | 100×250mm | | | filler: C18, 10µm | | |
|---|---|---|---|---|---|---|---|---|
| Mobile phase A: methanol | | | | Mobile phase B: 5‰ acetic acid/water | | | | |
| Gradient conditions | Time (min) | Concentration of mobile phase A | | | Concentration of mobile phase B | | Detection wavelength λ (nm) | Flow rate V (L/min) |
| | 0 | 70 | | | 30 | | 260 | 1.8 |
| | 30 | 70 | | | 30 | | 260 | 1.8 |
| | 100 | 85 | | | 15 | | 260 | 1.8 |

| | | | | | | | | |
|---|---|---|---|---|---|---|---|---|
| Eluents were fractionally collected to give the compound 61502-1r (1.1g) and 61502-1s (1.3g). | | | | | | | | |

### Example 14 Preparation of the compound Rₚ-1 (Reference)

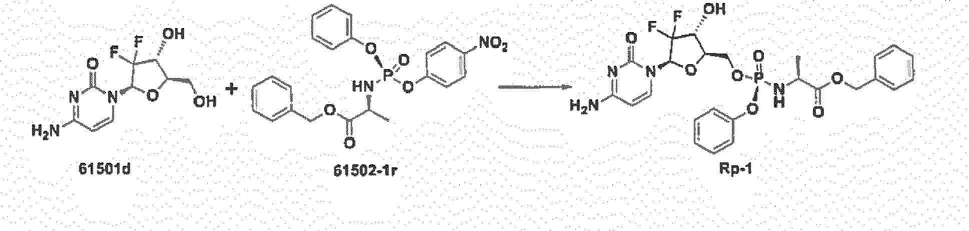

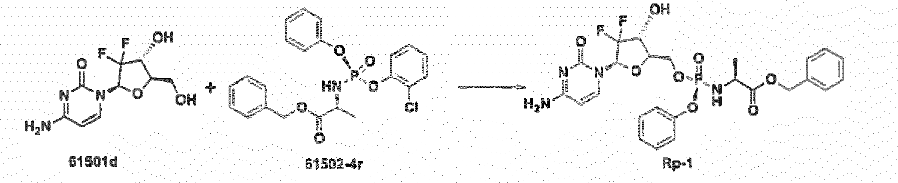

The compound Rᵣ-1 was prepared using the compound 61502-1r or 61502-4r instead of the compound 61501e by a method similar to Example 12. The HPLC purities are 98.9% and 99.1%, respectively. ³¹P-NMR (202MHz, MeOD): δ_{P} 3.81; ¹H-NMR (500MHz, MeOD): δ_{H}: 7.56, 7.52 (2d,J=7.5Hz, 1H,H-6), 7.38-7.33 (m, 7H, ArH), 7.26-7.19 (m, 3H, ArH), 6.25 (apparent q, J=7.5Hz, 1H, H-1'), 5.88,5.84 (2×d, J=7.5Hz, 1H, H-5), 5.18-5.12 (m, 2H, OCH₂Ph), 4.49-4.42 (m, 1H, H-5'), 4.38-4.31 (m, 1H, H-5'), 4.25-4.18 (m, 1H, H-3'), 4.07-4.01 (m, 2H,H-4', CHCH₃), 1.38 (apparent t, J=8.5Hz,3H,CHCH₃).

### Example 15 Preparation of the compound Sₚ-1

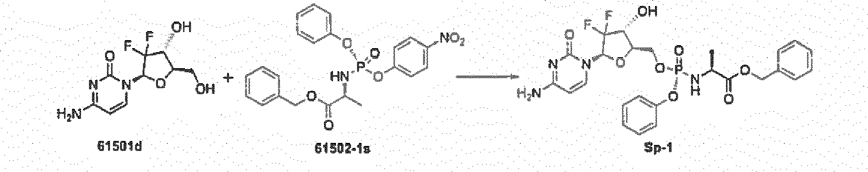

(1) Preparation of the compound Sₚ-1: To a solution of the compound 61501d (500mg) in tetrahydrofuran (10ml), a tert-butylmagnesium chloride solution (1.0mol/L, 5ml) was added at 0°C. After the mixture was stirred and reacted for 1h, 1g of the compound 61502-1s was added, and the mixture was stirred at room temperature until the reaction was complete. 30ml of water was added to the mixture, and the aqueous phase was extracted three times with ethyl acetate, each time with 20ml of ethyl acetate. The ethyl acetate phases were combined, washed with saline, dried over anhydrous sodium sulfate, and the solvent was removed. The compound Sₚ-1 (186mg) was obtained by purification via silica gel column chromatography (mobile phase: methanol/dichloromethane (the proportion of methanol increased from 2.5% to 5%), gradient elution), HPLC purity: 99.4%. ³¹P-NMR (202MHz,MeOD): δP 3.64; ¹H-NMR (500MHz, MeOD): δH: 7.56,7.52 (2d,J=7.5Hz, 1H,H-6), 7.38-7.33 (m, 7H, ArH), 7.26-7.19 (m, 3H, ArH), 6.25 (apparent q, J=7.5Hz, 1H, H-1'), 5.88,5.84 (2×d, J=7.5Hz, 1H, H-5), 5.18-5.12 (m, 2H, OCH₂Ph), 4.49-4.42 (m, 1H, H-5'), 4.38-4.31 (m, 1H, H-5'), 4.25-4.18 (m, 1H, H-3'), 4.07-4.01 (m, 2H,H-4', CHCH₃), 1.38 (apparent t, J=8.5Hz,3H,CHCH₃).

### Example 16 Preparation of the compound 61502-4, the compound 61502-4s and the compound 61502-4r

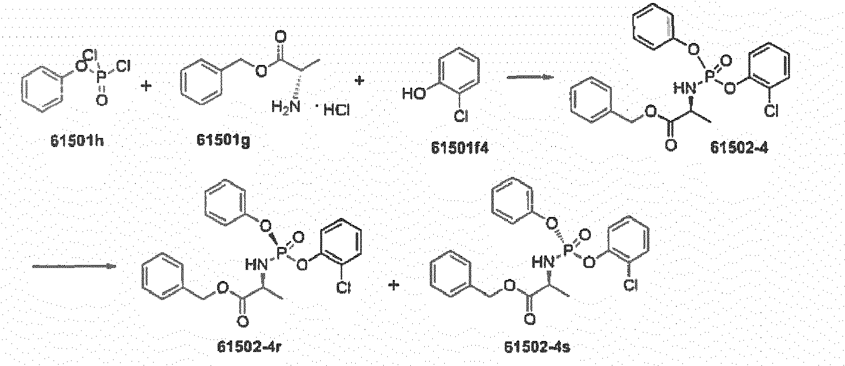

At -80°C, to a solution of 61501h (2g) in dichloromethane (10ml), 61501g (2.1g) was added, and then 2g of triethylamine (diluted with 5ml of dichloromethane) was added. The mixture was stirred overnight at room temperature. To the mixture, 61501f4 was added, and then 2g of triethylamine (diluted with 5ml of dichloromethane) was added. The mixture was stirred at room temperature for 4 h. The solvent was directly removed from the mixture, and the residue was dissolved in 50ml of ethyl acetate and 100ml of water. After the ethyl acetate was separated, the aqueous phase was washed twice with ethyl acetate, each time with 30ml of ethyl acetate. The ethyl acetate phases were combined, washed with saline, and dried over anhydrous sodium sulfate. Distilling off ethyl acetate resulted in the target compound 61502-4, which was used directly in the subsequent purification.

The compound 61502-4 (7.8g) was dissolved with 50ml of methanol, and prepared and purified through a preparative column with C18 filler (10µm) as the stationary phase. The preparation method was as the following Table 1: methanol and 5‰ acetic acid/water served as the mobile phases, the proportion of methanol increased from 70% to 80%, gradient elution.

**Table 1: Preparation Method**

| Preparative column: 100DAC | | | 100×250mm | | | filler: C18, 10µm | | |
|---|---|---|---|---|---|---|---|---|
| Mobile phase A: methanol | | | | Mobile phase B: 5‰ acetic acid/water | | | | |
| Gradient conditions | Time (min) | Concentration of mobile phase A | | | Concentration of mobile phase B | | Detection wavelength λ (nm) | Flow rate V (L/min) |
| | 0 | 70 | | | 30 | | 260 | 1.8 |
| | 30 | 70 | | | 30 | | 260 | 1.8 |
| | 100 | 85 | | | 15 | | 260 | 1.8 |

| | | | | | | | | |
|---|---|---|---|---|---|---|---|---|
| Eluents were fractionally collected to give the compound 61502-4r (1.05g) and 61502-4s (1.1g). | | | | | | | | |

### Example 17 Preparation of the compound Sₚ-1

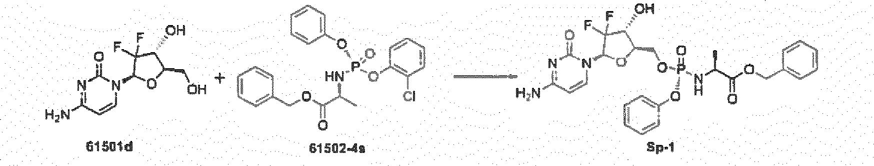

(1) Preparation of the compound Sₚ-1: To a solution of the compound 61501d (500mg) in tetrahydrofuran (10ml), a tert-butylmagnesium chloride solution (1.0mol/L, 5ml) was added at 0°C. After the mixture was stirred and reacted for 1h, 1g of the compound 61502-4s was added, and the mixture was stirred at room temperature until the reaction was complete. 30ml of water was added to the mixture, and the aqueous phase was extracted three times with ethyl acetate, each time with 20ml of ethyl acetate. The ethyl acetate phases were combined, washed with saline, dried over anhydrous sodium sulfate, and the solvent was removed. The compound Sₚ-1 (203mg) was obtained by purification via silica gel column chromatography (mobile phase: methanol/dichloromethane (the proportion of methanol increased from 2.5% to 5%), gradient elution), HPLC purity: 98.3%. ³¹P-NMR (202MHz,MeOD): δP 3.64; ¹H-NMR(500MHz, MeOD): δH: 7.56,7.52 (2d,J=7.5Hz, 1H,H-6), 7.38-7.33 (m, 7H, ArH), 7.26-7.19 (m, 3H, ArH), 6.25 (apparent q, J=7.5Hz, 1H, H-1'), 5.88,5.84 (2×d, J=7.5Hz, 1H, H-5), 5.18-5.12 (m, 2H, OCH₂Ph), 4.49-4.42 (m, 1H, H-5'), 4.38-4.31 (m, 1H, H-5'), 4.25-4.18 (m, 1H, H-3'), 4.07-4.01 (m, 2H,H-4', CHCH₃), 1.38 (apparent t, J=8.5Hz,3H,CHCH₃).

### Example 18 Preparation of the compound Sₚ-1

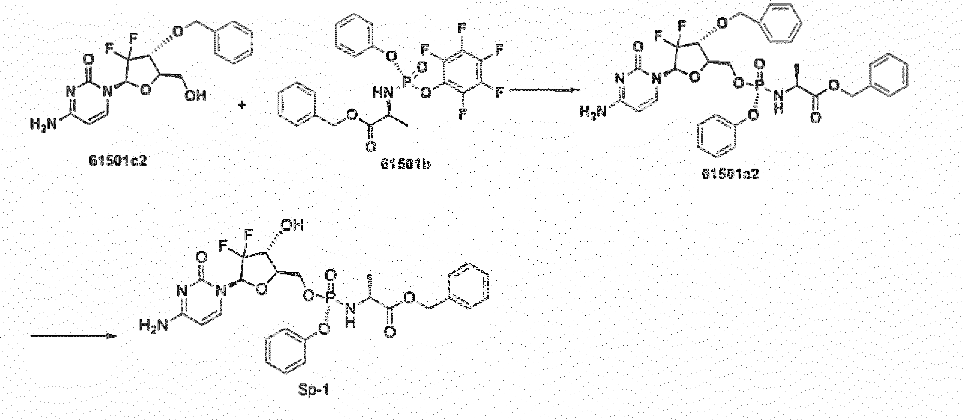

(1) Preparation of the compound 61501a2: To a solution of the compound 61501c2 (700mg) in tetrahydrofuran (10ml) was added a tert-butylmagnesium chloride solution (1.0mol/L, 7ml) at 0°C, After the mixture was stirred and reacted for 1h, the compound 61501b (2g) was added. The mixture was stirred at room temperature until the reaction was complete. To the mixture, 50ml of water was added, and the aqueous phase was extracted three times with ethyl acetate, each time with 50ml of ethyl acetate. The ethyl acetate phases were combined, washed with saline, dried over anhydrous sodium sulfate, and the solvent was removed. 700mg of the compound 61501a2 was obtained by purification via silica gel column chromatography (mobile phase: methanol/dichloromethane (the proportion of methanol increased from 2.5% to 5%), gradient elution) with a yield of 55%.
(2) Preparation of the compound Sₚ-1: Pd/C (150mg) was added to a solution of the compound 61501a2 (700mg) in 5ml of methanol at room temperature. After the reaction system was purged with hydrogen gas, the reaction proceeded at room temperature with the pressure maintained at 0.4bar until it was complete. The reaction solution was filtered directly. After the solvent was removed from the filtrate, the compound Sₚ-1 (350mg) was obtained by purification via silica gel column chromatography (mobile phase: methanol/dichloromethane (the proportion of methanol increased from 2.5% to 10%), gradient elution), HPLC purity: 99.3%. ³¹P-NMR (202MHz,MeOD): δₚ 3.64. ¹H-NMR (500MHz,MeOD): δ_{H}: 7.56,7.52 (2d,J=7.5Hz, 1H,H-6), 7.38-7.33 (m,7H,ArH), 7.26-7.19 (m,3H,ArH), 6.25 (apparent q, J=7.5Hz,1H,H-1'), 5.88,5.84 (2xd,J=7.5Hz,1H,1H-5), 5.18-5.12 (m,2H,OCH₂Ph), 4.49-4.42 (m,1H,H-5'), 4.38-4.31 (m,1H,H-5'), 4.25-4.18 (m,1H,H-3'), 4.07-4.01 (m,2H,H-4',CHCH₃), 1.38 (apparent t,J=8.5Hz,3H,CHCH₃).

### Example 19 Preparation of the compound Sₚ-1

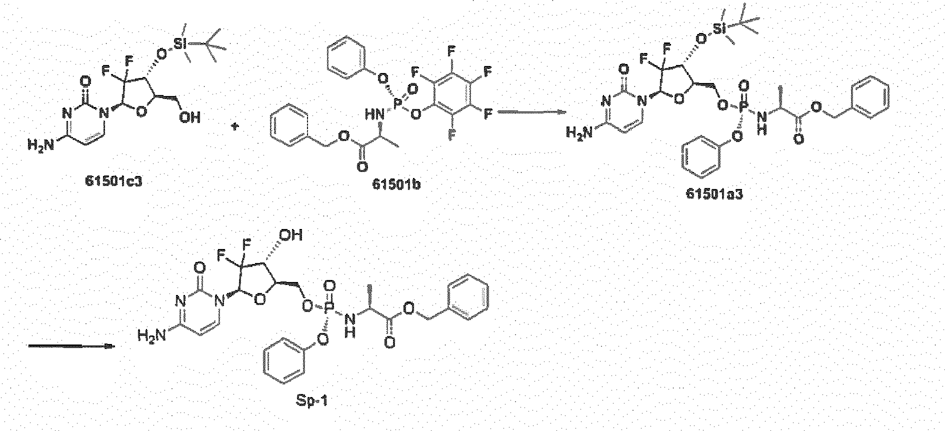

(1) Preparation of the compound 61501a3: To a solution of the compound 61501c3 (700mg) in tetrahydrofuran (10ml), a tert-butylmagnesium chloride solution (1.0mol/L, 8ml) was added at 0°C. After the mixture was stirred and reacted for 1 h, the compound 61501b (2g) was added, and the mixture was stirred at room temperature until the reaction was complete. To the mixture, 50ml of water was added, and the aqueous phase was extracted three times with ethyl acetate, each time with 50ml of ethyl acetate. The ethyl acetate phases were combined, washed with saline, dried over anhydrous sodium sulfate, and the solvent was removed. 600mg of the compound 61501a3 was obtained by purification via silica gel column chromatography (mobile phase: methanol/dichloromethane (the proportion of methanol increased from 2.5% to 5%), gradient elution) .
(2) Preparation of the compound Sₚ-1: At 0°C, to a solution of the compound 61501a3 (600mg) in methanol (5ml), 1mol/L hydrochloric acid (1ml) was added. The reaction proceeded at 0°C until it was complete. The solvent was directly removed from the reaction solution, the residue was dissolved with 50ml of ethyl acetate, washed with saturated saline, and dried over anhydrous sodium sulfate. After the solvent was removed, the compound Sₚ-1 (300mg) was obtained by purification via silica get column chromatography (mobile phase: methanol/dichloromethane (the proportion of methanol increased from 2.5% to 10%), HPLC purity: 99.5%. ³¹P-NMR (202MHz, MeOD): δ_{P} 3.64. ¹H-NMR(500MHz, MeOD): δ_{H}: 7.56,7.52 (2d,J=7.5Hz,1H,H-6), 7.38-7.33 (m,7H,ArH), 7.26-7.19 (m,3H,ArH), 6.25 (apparent q, J=7.5Hz,1H,H-1'), 5.88,5.84 (2×d,J=7.5Hz,1H,H-5), 5.18-5.12 (m,2H,OCH₂Ph), 4.49-4.42 (m,1H,H-5'), 4.38-4.31 (m,1H,H-5'), 4.25-4.18 (m,1H,H-3'), 4.07-4.01 (m,2H,H-4',CHCH₃), 1.38 (apparent t,J=8.5Hz,3H,CFCH₃).

### Example 20 Preparation of the compound Sₚ-1

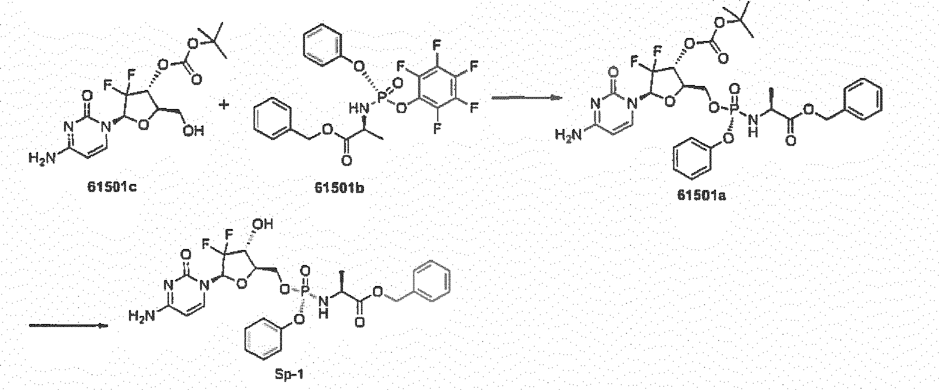

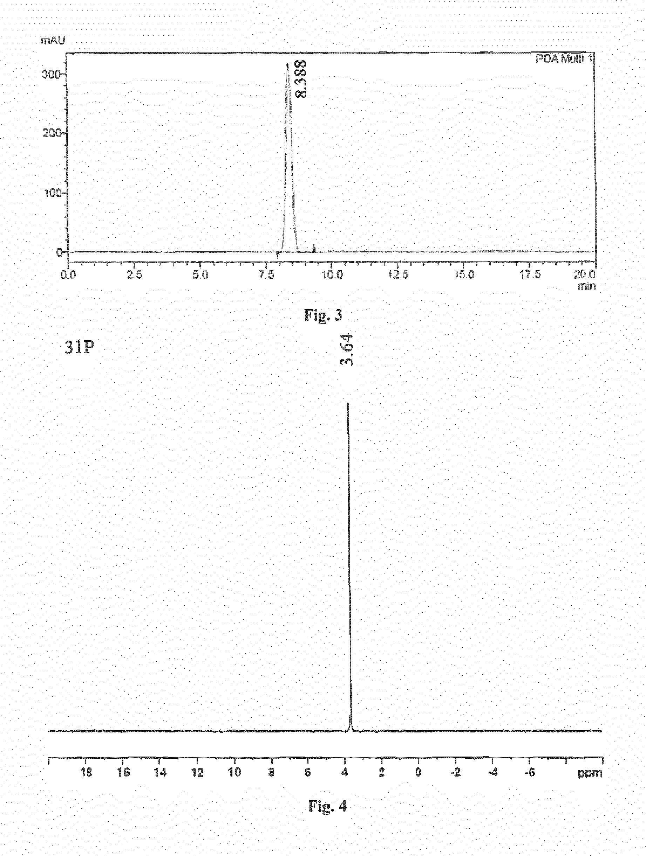

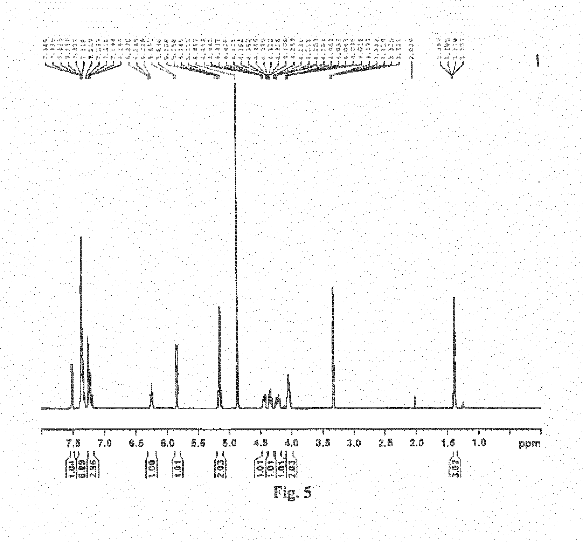

(1) Preparation of the compound 61501a: To a solution of the compound 61501c (2g) in tetrahydrofuran (10ml) was added a tert-butylmagnesium chloride solution (1.0mol/L, 16ml) at 0°C. After the mixture was stirred and reacted for 1 h, the compound 61501b (5g) was added. The mixture was stirred at room temperature until the reaction was complete. To the mixture, 50ml of water was added, and the aqueous phase was extracted three times with ethyl acetate, each time with 100ml of ethyl acetate. The ethyl acetate phases were combined, washed with saline, dried over anhydrous sodium sulfate, and the solvent was removed. 2.8g of the compound 61501a was obtained by purification via silica gel column chromatography (eluting reagents: methanol/dichloromethane 2.5% to 5%) with a yield of 77%, HPLC purity: 93% .
(2) Preparation of the compound Sₚ-1: 6ml of trifluoroacetic acid was added to a solution of the compound 61501a (2g) in dichloromethane (10ml) at 0°C. The mixture was stirred with the temperature kept unchanged until the reaction was complete. After the solvent was removed from the mixture, 50ml of sodium bicarbonate solution was added, and the resulted mixture was extracted three times with ethyl acetate, each time with 50ml of ethyl acetate. The ethyl acetate phases were combined, washed with saline, and dried over anhydrous sodium sulfate. After the solvent was removed, the compound Sₚ-1 (700mg) was obtained via silica gel column chromatography (mobile phase: methanol/dichloromethane (the proportion of methanol increased from 2.5% to 5%), gradient elution), HPLC purity: 100% (as shown in Fig. 3). ³¹P-NMR (202MHz,MeOD): δp 3.64 (as shown in Fig. 4); ¹H-NMR(500MHz,MeOD): δ_{H}: 7.56,7.52 (2d,J=7.5Hz,1H,H-6), 7.38-7.33 (m,7H,ArH), 7.26-7.19 (m,3H,ArH), 6.25 (apparent q, J=7.5Hz,11-1,H-1'), 5.88,5.84 (2×d,J=7.5Hz,1H,H-5), 5.18-5.12 (m,2H,OCH₂Ph), 4.49-4.42 (m,1H,H-5'), 4.38-4.31 (m,1H,H-5'), 4.25-4.18 (m,1H,H-3'), 4.07-4.01 (m,2H,H-4',CHCH₃), 1.38 (apparent t,J=8.5Hz,3H,CHCH₃) (as shown in Fig. 5).

### Example 21 Preparation of the compound Sₚ-1

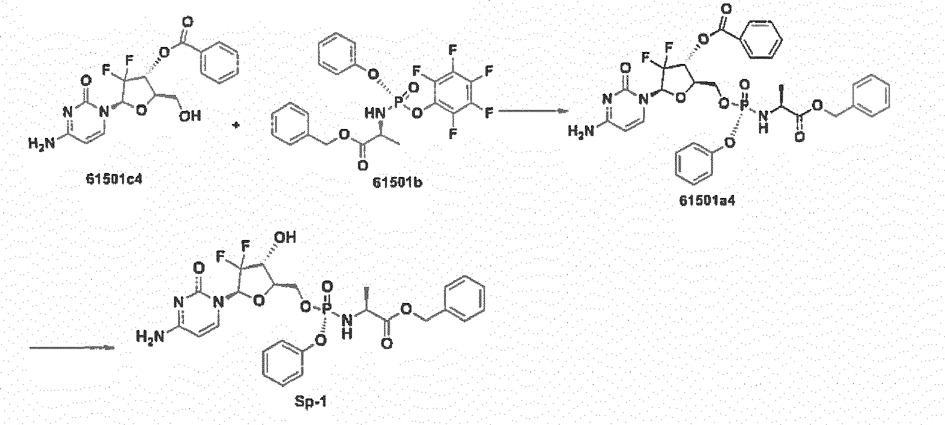

(1) Preparation of the compound 61501a4: To a solution of the compound 61501c4 (600mg) in tetrahydrofuran (10ml), a tert-butylmagnesium chloride solution (1.0mol/L, 7ml) was added at 0°C. After the mixture was stirred and reacted for 1 h, the compound 61501b (1.8g) was added, and the mixture was stirred at room temperature until the reaction was complete. 50ml of water was added to the mixture, the aqueous phase was extracted three times with ethyl acetate, each time with 50ml of ethyl acetate. The ethyl acetate phases were combined, washed with saline, dried over anhydrous sodium sulfate, and the solvent was removed. 550mg of the compound (61501a4) was obtained by purification via silica gel column chromatography (mobile phase: methanol/dichloromethane (the proportion of methanol increased from 2.5% to 5%), gradient elution).
(2) Preparation of the compound Sₚ-1: To a solution of the compound 61501a4 (500mg) in methanol (5ml), 1.5ml of 7mol/L methanolic ammonia solution was added at room temperature. The reaction proceeded at room temperature until it was complete. The solvent was directly removed from the reaction solution, the residue was dissolved with 50ml of ethyl acetate, washed with saturated saline, dried over anhydrous sodium sulfate, and then the solvent was removed. The compound Sₚ-1 (300mg) was obtained via silica gel column chromatography (mobile phase: methanol/dichloromethane (the proportion of methanol increased from 2.5% to 5%), gradient elution), HPLC purity: 99.5%. ³¹P-NMR (202MHz,MeOD): δₚ 3.64. ¹H-NMR(500MHz,MeOD): δ_{H}: 7.56,7.52 (2d,J=7.5Hz,1H,H-6), 7.38-7.33 (m,7H,ArH), 7.26-7.19 (m,3H,ArH), 6.25 (apparent q, J=7.5Hz,1H,H-1'), 5.88,5.84 (2×d,J=7.5Hz,1H,H-5}, 5.18-5.12 (m,2H,OCH₂Ph), 4.49-4.42 (m,1H,H-5'), 4.38-4.31 (m,1H,H-5'), 4.25-4.18 (m, 1H,H-3'), 4.07-4.01 (m,2H,H-4',CHCH₃), 1.38 (apparent t,J=8.5Hz,3H,CHCH₃).

### Example 22 Determination of the configuration of the compound Sₚ-1

In order to further determine the configuration of the compound Sₚ-1 of the present disclosure, the present inventors used the compound Sₚ-1 obtained by the methods provided by the present disclosure, e.g., the methods of Examples 15, 17, and 18 to 21, to grow the single crystal of the compound. For instance, a rod-shaped single crystal was obtained by adding the compound Sₚ-1 to a solvent composed of, for example, a lower alcohol and water, and the temperature of the system was reduced slowly from 30°C-50°C to 0°C-5°C. Preferably, said lower alcohol is a C₁₋₃ alcohol (e.g., methanol, ethanol, propanol or isopropanol). More preferably, said lower alcohol is ethanol. In the solvent system for the cultivation of single crystal, the amount ratio of the lower alcohol to that of water is preferably 1:3 (v/v), and the single crystal analysis was performed.

In order to contribute to the identification of the new crystal form, the present disclosure provided the data of X-ray diffraction analysis and the conditions adopted to achieve these data, which were as follows:

| Temperature | 123(2) K |
|---|---|
| Wavelength | 0.71073Å |
| Crystal system, space group | Monoclinic, *P2₁* |
| Unit cell dimensions | a=11.3607(19)Å |
| | b=34.841(5) Å |
| | c=15,118(2) Å |
| | α=90° |
| | β=111.857(4)° |
| | γ=90° |
| Volume | 5553.8(15)Å³ |
| Z | 8 |
| Calculated density | 1.388 Mg/m³ |
| Absorption coefficient | 0.166 mm⁻¹ |
| F(000) | 2416 |
| Crystal size | 2.26×0.26×0.23mm |
| θ range of data collection | 1.97° to 27.63° |
| Reflections collected / unique | 123782/13011[R(int) =0.1357] |
| Completeness | 98.9% |
| Refinement method | Full-matrix least-squares on F² |
| Data/restraints/parameters | 13011/3/1403 |
| *Goodness-of-fit on F²* | 1.858 |
| Final R indices [(1 > 2sigma(I)] | R₁=0.1853, wR₂=0.4314 |
| Largest diff. peak and hole | 1.575 and -1.177e.A⁻³ |

The diffraction data of the single crystal was collected at 123(2) K using a Bruker D8 ADVANCE diffractometer for single crystal(MoKa, λ = 0.71073Å).

### Preparation of the compound Sₚ-1 in crystalline form

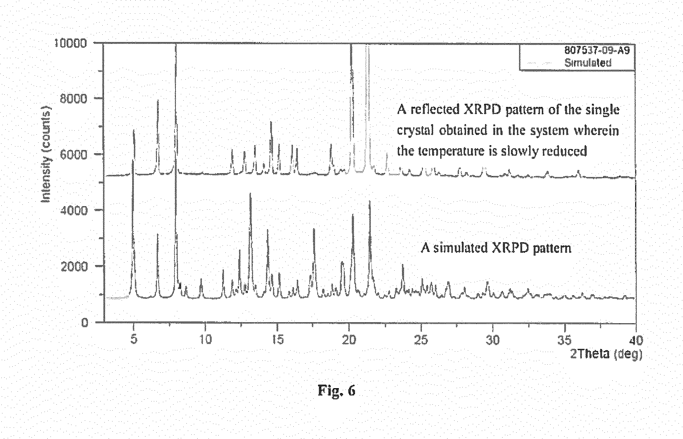

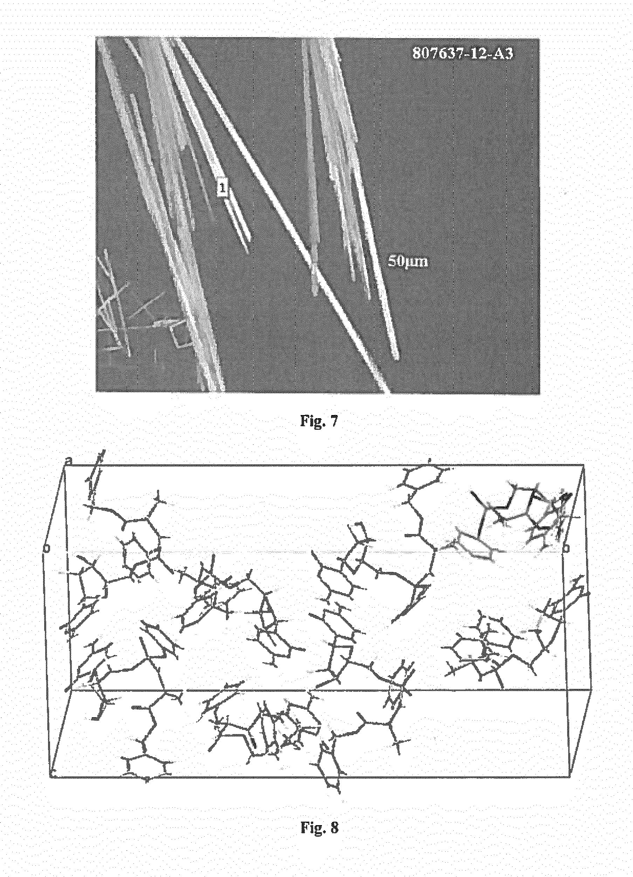

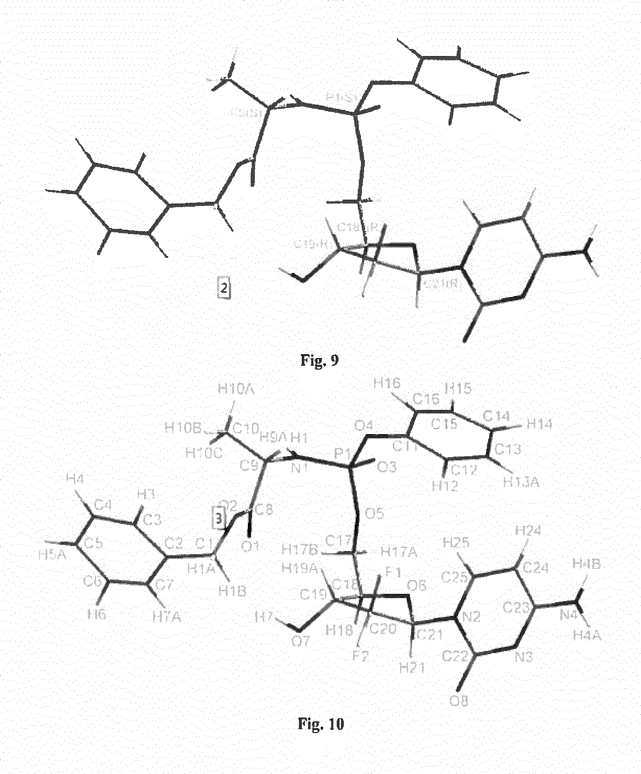

The compound Sₚ-1 prepared in Example 15 was added to a system of ethanol/water (1/3, v/v), and the temperature was slowly reduced from 50°C to 5°C at a rate of 0.01°C/min, resulting in a rod-shaped single crystal (as shown in Fig. 7). The reflected XRPD pattern of the single crystal was as shown in Fig. 6, wherein the unit cell dimensions of the single crystal was as shown in Fig. 8. The absolute configuration of the molecule of the compound Sₚ-1 was determined by the single crystal analysis to be as follows: phosphorus atom P1 (S), C9 (S), C18 (R), C19 (R), C21 (R) (as shown in Figs. 9 and 10).

## Claims

1. A method of preparing a composition, comprising the compound Sₚ-1 in an amount of at least about 95% by weight 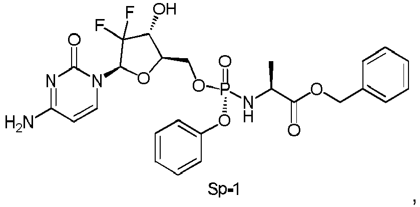 wherein the method comprises reacting a compound 61501c with a compound 61501b to produce a compound 61501a: 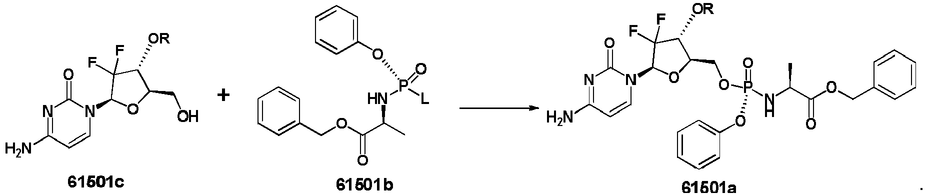
wherein R is H or a hydroxyl protecting group; L is halogen, aryloxide, benzenesulfonate group, camphorsulfonate group, or aryloxide substituted with at least one electron withdrawing group;
optionally, when R is not H, the hydroxyl protecting group of the compound 61501a is deprotected to give the compound Sₚ-1.

2. The method according to claim 1, wherein the electron withdrawing group is nitro or halogen.

3. The method according to claim 1, wherein L is nitrophenoxide, p-chlorophenoxide, o-chlorophenoxide, 2,4-dinitrophenoxide or pentafluorophenoxide.

4. The method according to any one of claims 1 to 3, wherein the hydroxyl protecting group R is alkylsilyl, alkyl or substituted alkyl, acyl or substituted acyl, alkoxycarbonyl or substituted alkoxycarbonyl.

5. The method according to any one of claims 1 to 4, wherein the hydroxyl protecting group R is tetrahydropyranyl, benzyl, p-methylbenzyl, acetyl, propionyl, butyryl, benzoyl, *tert*-butyloxy carbonyl (Boc), benzyloxycarbonyl (Cbz), 9-fluorenylmethoxycarbonyl (Fmoc), *tert*butyldimethylsilyl, trimethylsilyl or dimethylphenylsilyl.

6. The method according to claim 1, wherein R is H or *tert*-butyloxy carbonyl (Boc), and L is pentafluorophenoxide.

7. The method according to claim 1, wherein the preparation method of the compound 61501b comprises the following steps: 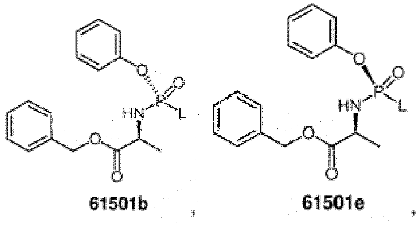
(1) reacting a compound (PhO)P(O)(L')₂ and alanine benzyl ester in the presence of a first alkali to obtain an (L')P(O)(PhO)(Ala-CH₂Ph);
(2) reacting the (L')P(O)(PhO)(Ala-CH₂Ph) with a phenol in the presence of a second alkali to obtain a mixture comprising the compound 61501b and the compound 61501e;
(3) subjecting the mixture comprising the compound 61501b and the compound 61501e in step (2) to extraction, chromatographic separation or crystallization to obtain the compound 61501b;
wherein L is aryloxide, benzenesulfonate group, camphorsulfonate group, or aryloxide substituted with at least one electron withdrawing group, and L' is a leaving group independent of L.

8. The method according to claim 7, wherein in step (3), the mixture comprising the compound 61501b and the compound 61501e is dissolved or suspended into a solvent, and an anti-solvent is added to crystalize and obtain the compound 61501b.

9. The method according to any of claims 1 to 8, wherein the composition comprises the compound Sₚ-1 in an amount of at least about 99% by weight.

## Patentansprüche

1. Verfahren zur Herstellung einer Zusammensetzung, die die Verbindung Sₚ-1 in einer Menge von mindestens etwa 95 Gew.-% umfasst 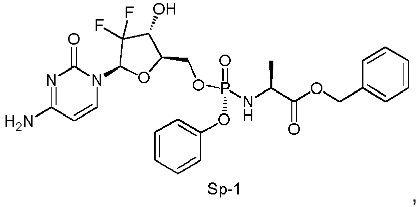 wobei das Verfahren das Umsetzen einer Verbindung 61501c mit einer Verbindung 61501b zur Herstellung einer Verbindung 61501a umfasst: 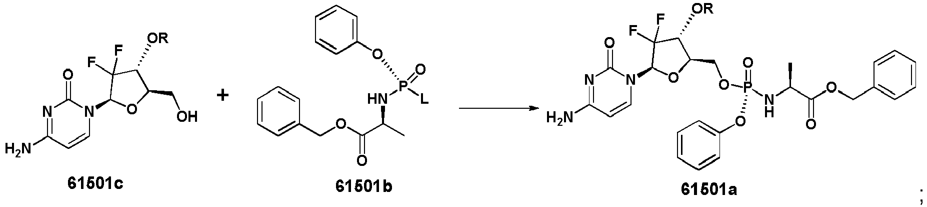
wobei R H oder eine Hydroxylschutzgruppe ist; L Halogen, Aryloxid, eine Benzolsulfonatgruppe, eine Camphersulfonatgruppe oder ein mit mindestens einer elektronenziehenden Gruppe substituiertes Aryloxid ist;
gegebenenfalls, wenn R nicht H ist, die Hydroxylschutzgruppe der Verbindung 61501a entschützt wird, um die Verbindung Sₚ-1 zu erhalten.

2. Das Verfahren nach Anspruch 1, wobei die elektronenziehende Gruppe Nitro oder Halogen ist.

3. Das Verfahren nach Anspruch 1, wobei L Nitrophenoxid, p-Chlorphenoxid, o-Chlorphenoxid, 2,4-Dinitrophenoxid oder Pentafluorphenoxid ist.

4. Das Verfahren nach einem der Ansprüche 1 bis 3, wobei die Hydroxylschutzgruppe R Alkylsilyl, Alkyl oder substituiertes Alkyl, Acyl oder substituiertes Acyl, Alkoxycarbonyl oder substituiertes Alkoxycarbonyl ist.

5. Das Verfahren nach einem der Ansprüche 1 bis 4, wobei die Hydroxylschutzgruppe R Tetrahydropyranyl, Benzyl, p-Methylbenzyl, Acetyl, Propionyl, Butyryl, Benzoyl, *tert-*Butyloxycarbonyl (Boc), Benzyloxycarbonyl (Cbz), 9-Fluorenylmethoxycarbonyl (Fmoc), *tert-*Butyldimethylsilyl, Trimethylsilyl oder Dimethylphenylsilyl ist.

6. Das Verfahren nach Anspruch 1, wobei R H oder *tert*-Butyloxycarbonyl (Boc) ist und L Pentafluorphenoxid ist.

7. Das Verfahren nach Anspruch 1, wobei das Herstellungsverfahren der Verbindung 61501b die folgenden Schritte umfasst: 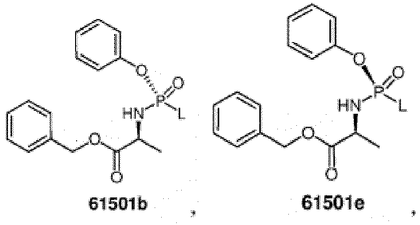
(1) Umsetzen einer Verbindung (PhO)P(O)(L')₂ und eines Alaninbenzylesters in Gegenwart eines ersten Alkalis, um ein (L')P(O)(PhO)(Ala-CH₂Ph) zu erhalten;
(2) Umsetzen des (L')P(O)(PhO)(Ala-CH₂Ph) mit einem Phenol in Gegenwart eines zweiten Alkalis, um eine Mischung zu erhalten, die die Verbindung 61501b und die Verbindung 61501e umfasst;
(3) Unterziehen der Mischung, die die Verbindung 61501b und die Verbindung 61501e in Schritt (2) umfasst, einer Extraktion, chromatographischen Trennung oder Kristallisation, um die Verbindung 61501b zu erhalten;
wobei L ein Aryloxid, eine Benzolsulfonatgruppe, eine Camphersulfonatgruppe oder ein mit mindestens einer elektronenziehenden Gruppe substituiertes Aryloxid ist, und L' eine von L unabhängige Abgangsgruppe ist.

8. Das Verfahren nach Anspruch 7, wobei in Schritt (3) das Gemisch, das die Verbindung 61501b und die Verbindung 61501e umfasst, in einem Lösungsmittel gelöst oder suspendiert wird und ein Antilösungsmittel zugegeben wird, um die Verbindung 61501b zu kristallisieren und zu erhalten.

9. Das Verfahren nach einem der Ansprüche 1 bis 8, wobei die Zusammensetzung die Verbindung Sₚ-1 in einer Menge von mindestens etwa 99 Gew.-% umfasst.

## Revendications

1. Procédé de préparation d'une composition, comprenant le composé Sₚ-1 à hauteur d'au moins environ 95 % en poids 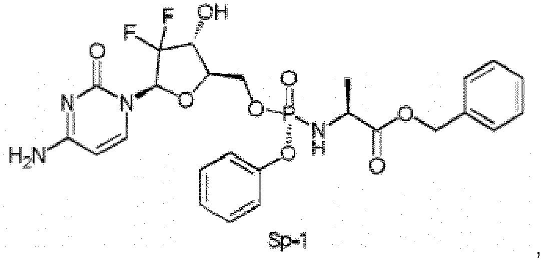
le procédé comprenant la réaction d'un composé 61501c avec un composé 61501b pour produire un composé 61501a : 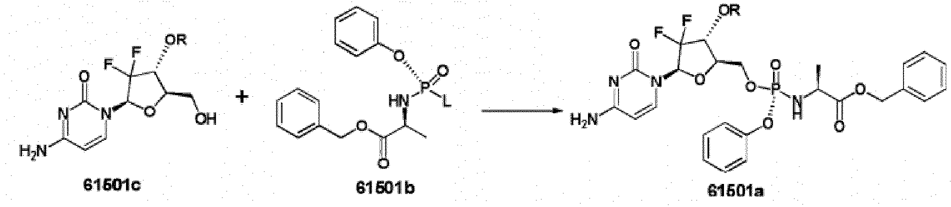
dans lesquels R est H ou un groupe protecteur d'hydroxyle ; L est un halogène, un aryloxyde, un groupe benzènesulfonate, un groupe camphorsulfonate, ou un aryloxyde substitué par au moins un groupe attracteur d'électron ;
éventuellement, lorsque R n'est pas H, le groupe protecteur d'hydroxyle du composé 61501a est déprotégé pour donner le composé Sₚ-1.

2. Procédé selon la revendication 1, dans lequel le groupe attracteur d'électron est un groupe nitro ou un halogène.

3. Procédé selon la revendication 1, dans lequel L est un nitrophénoxyde, p-chlorophénoxyde, o-chlorophénoxyde, 2,4-dinitrophénoxyde ou pentafluorophénoxyde.

4. Procédé selon l'une quelconque des revendications 1 à 3, dans lequel le groupe protecteur d'hydroxyle R est un alkylsilyle, un alkyle ou un alkyle substitué, un acyle ou un acyle substitué, un alcoxycarbonyle ou un alcoxycarbonyle substitué.

5. Procédé selon l'une quelconque des revendications 1 à 4, dans lequel le groupe protecteur d'hydroxyle R est un tétrahydropyranyle, benzyle, p-méthylben-zyle, acétyle, propionyle, butyryle, benzoyle, *tert*-butyloxycarbonyle (Boc), benzy-loxycarbonyle (Cbz), 9-fluorénylméthoxycarbonlyle (Fmoc), *tert*-butyldiméthylsilyle, triméthylsilyle ou diméthylphénylsilyle.

6. Procédé selon la revendication 1, dans lequel R est H ou le tert-butyloxycarbonyle (Boc), et L est le pentafluorophénoxyde.

7. Procédé selon la revendication 1, dans lequel le procédé de préparation du composé 61501b comprend les étapes suivantes : 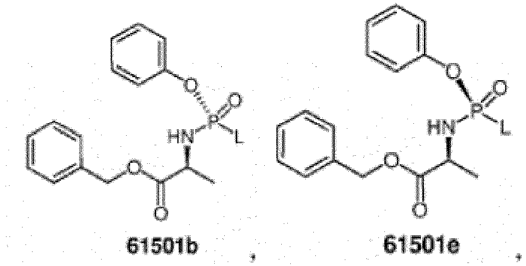
(1) réaction d'un composé (PhO)P(O)(L')₂ et d'un ester benzylique d'alanine en présence d'un premier alcali pour obtenir du (L')P(O)(PhO)(Ala-CH₂Ph) ;
(2) réaction du (L')P(O)(PhO)(Ala-CH₂Ph) avec un phénol en présence d'un second alcali pour obtenir un mélange comprenant le composé 61501b et le composé 61501e ;
(3) soumission du mélange comprenant le composé 61501b et le composé 61501e de l'étape (2) à une extraction, une séparation chromatographique ou une cristallisation pour obtenir le composé 61501b ;
L étant un aryloxyde, un groupe benzènesulfonate, un groupe camphorsulfonate, ou un aryloxyde substitué par au moins un groupe attracteur d'électron, et L' étant un groupe labile indépendant de L.

8. Procédé selon la revendication 7, où à l'étape (3), le mélange comprenant le composé 61501b et le composé 61501e est dissous ou mis en suspension dans un solvant, et un anti-solvant est ajouté pour cristalliser et obtenir le composé 61501b.

9. Procédé selon l'une quelconque des revendications 1 à 8, la composition comprenant le composé Sₚ-1 à hauteur d'au moins environ 99 % en poids.
